(19) **Europäisches Patentamt European Patent Office Office européen des brevets**

(11) **EP 4 656 658 A1**

(12) **EUROPEAN PATENT APPLICATION**
published in accordance with Art. 153(4) EPC

(43) Date of publication:
03.12.2025 Bulletin 2025/49

(21) Application number: 24919140.4

(22) Date of filing: 13.04.2024

(51) International Patent Classification (IPC):
C07K 16/28 (2006.01)     A61P 35/00 (2006.01)
A61K 39/00 (2006.01)

(86) International application number:
PCT/KR2024/004990

(87) International publication number:
WO 2025/216346 (16.10.2025 Gazette 2025/42)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Immuneoncia Therapeutics, Inc.
Yongin-si, Gyeonggi-do 17084 (KR)

(72) Inventors:
• KIM, Heung Tae
Yongin-si, Gyeonggi-do 17084 (KR)

• LEE, Sung Young
Yongin-si, Gyeonggi-do 17084 (KR)
• KIM, Sung Ho
Yongin-si, Gyeonggi-do 17084 (KR)

(74) Representative: Ullrich & Naumann PartG mbB
Schneidmühlstrasse 21
69115 Heidelberg (DE)

Remarks:
The complete document including Reference
Table(s) and the Sequence Listing(s) can be
downloaded from the EPO website

(54) **NEOADJUVANT THERAPY USING PD-L1 INHIBITOR FOR TREATING CANCER**

(57) The present disclosure relates to a method for treating a tumor or inhibiting tumor proliferation, comprising administering to a cancer patient in need thereof a therapeutically effective amount of a programmed death-ligand 1 (PD-L1) inhibitor (for example, an anti-PD-L1 antibody) as neoadjuvant therapy, followed by surgical resection. In particular, the present disclosure relates to a method for administering a PD-L1 inhibitor as preoperative therapy to a patient with resectable upper gastrointestinal cancer, for example, resectable localized gastric cancer, esophageal cancer, or liver cancer. Administration of a PD-L1 inhibitor, in particular, an anti-PD-L1 antibody, has been shown to exhibit excellent therapeutic effects as preoperative therapy for patients with resectable localized gastric cancer, esophageal cancer, and liver cancer.

[FIG. 1]

EP 4 656 658 A1

## Description

### Technical Field

The present disclosure relates to a method for treating a tumor and inhibiting tumor proliferation, comprising administering to a cancer patient a therapeutically effective amount of a programmed death-ligand 1 (PD-L1) inhibitor, such as the anti-PD-L1 antibody IMC-001 or a bioequivalent thereof, as neoadjuvant therapy, followed by surgical resection. In particular, the present disclosure relates to a method for administering a PD-L1 inhibitor as preoperative therapy to a patient with surgically resectable upper gastrointestinal cancer, such as localized gastric cancer, esophageal cancer, or liver cancer.

Sequence Listing

This application includes a sequence listing that has been submitted electronically in XML format and is incorporated herein by reference in its entirety. A copy of the sequence listing, created on April 13, 2024, is named KC24060.xml and is 12.3 kilobytes in size.

### Background Art

Immune evasion has recently gained attention as one of the key hallmarks of cancer, and advancements in tumor immunology have begun to elucidate its underlying mechanisms. An immune checkpoint is a concept including immunosuppressive regulatory molecules that prevent immune cells from attacking self-cells. Tumor cells exploit this immune checkpoint as one of several mechanisms to evade immune surveillance, thereby creating an immunosuppressive environment within tumors.

A therapeutic strategy using cancer immunotherapy, which achieves anticancer effects by activating suppressed anti-tumor immune system, has attracted attention, and representative immune checkpoint inhibitors, such as CTLA-4 inhibitors or PD-1/PD-L1 inhibitors, have recently demonstrated improved survival rates in various cancers. As a result, cancer immunotherapy has become one of the standard cancer treatments and has presented a new paradigm in cancer treatment (Pardoll DM: The blockade of immune checkpoints in cancer immunotherapy. Nat Rev Cancer 12:252-64, 2012).

There are study results on lung cancer, indicating that immune checkpoint inhibitors exhibit a greater therapeutic effect in tumors with a high number of mutations, in particular, in a case where there is a high burden of clonal neoantigens, and that even in tumors with a high total number of mutations, PD-1 inhibitors exhibit a decreased therapeutic effect if there is a high burden of subclonal neoantigens (McGranahan N, Furness AJ, Rosenthal R, et al.: Clonal neoantigens elicit T cell immunoreactivity and sensitivity to immune checkpoint blockade. Science 351:1463-9, 2016).

In addition, considering that as a tumor evolves with cancer progression, the number of subclonal neoantigens increases and the immunosuppressive microenvironment progresses, immune checkpoint inhibitors are likely to exhibit a higher therapeutic effect on early-stage localized cancer than stage IV metastatic cancers (Jamal-Hanjani M, Quezada SA, Larkin J, et al.: Translational implications of tumor heterogeneity. Clin Cancer Res 21:1258-66, 2015; and Gil Del Alcazar CR, Huh SJ, Ekram MB, et al.: Immune Escape in Breast Cancer During In Situ to Invasive Carcinoma Transition. Cancer Discov 7:1098-1115, 2017).

Immune checkpoint inhibitors, such as pembrolizumab, nivolumab, and ipilimumab, have already been demonstrated to improve survival rates or reduce tumor size in patients with unresectable locally advanced or metastatic gastric cancer, esophageal cancer, and hepatocellular carcinoma, thereby demonstrating that therapeutic strategies using immune checkpoint inhibitors are effective in these cancers.

On the other hand, surgical resection with curative intent is a treatment option for resectable gastric cancer, esophageal cancer, and hepatocellular carcinoma. However, tumor recurrence is common after surgery, and early recurrences are also observed. Therefore, there is an urgent need for safe and effective therapies to treat surgically resectable cancers.

### Disclosure of Invention

### Technical Problem

The object of the present disclosure is to solve all of the above-mentioned problems of the prior art.

An object of the present disclosure is to provide a preoperative therapy, a method for treating cancer, a pharmaceutical composition for treating cancer, a kit, and a dosing regimen for a patient with surgically resectable upper gastrointestinal cancer.

The object of the present disclosure is not limited to the above-mentioned objects. The objects of the present disclosure will become clearer from the following description and may be realized by means and combinations thereof as set forth in the claims.

**Solution to Problem**

Representative configurations of the present disclosure to achieve the above-mentioned objects are as follows.

According to an aspect of the present disclosure, there is provided a pharmaceutical composition for treating a tumor or inhibiting tumor proliferation in a patient with upper gastrointestinal cancer, the composition comprising a therapeutically effective amount of a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2), wherein the composition is administered to the patient as neoadjuvant therapy prior to surgical resection of the tumor.

According to another aspect of the present disclosure, there is provided a method for treating a tumor or inhibiting tumor growth, comprising: (a) selecting a patient with upper gastrointestinal cancer; (b) administering a therapeutically effective amount of a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) to the patient; and (c) surgically resecting a tumor of the upper gastrointestinal cancer after step (b).

In an embodiment, the upper gastrointestinal cancer may include gastric cancer, esophageal cancer, or liver cancer.

In an embodiment, the anti-PD-L1 antibody may comprise a heavy chain variable region comprising HCDR1 of SEQ ID NO: 5, HCDR2 of SEQ ID NO: 6, and HCDR3 of SEQ ID NO: 7, and a light chain variable region comprising LCDR1 of SEQ ID NO: 8, LCDR2 of SEQ ID NO: 9, and LCDR3 of SEQ ID NO: 10.

In an embodiment, the light chain variable region of the anti-PD-L1 antibody may comprise or consist of the amino acid sequence of SEQ ID NO: 2, or an amino acid sequence that is at least 95% identical to SEQ ID NO: 2.

In an embodiment, the heavy chain variable region of the anti-PD-L1 antibody may comprise or consist of the amino acid sequence of SEQ ID NO: 1, or an amino acid sequence that is at least 95% identical to SEQ ID NO: 1.

In an embodiment, the anti-PD-L1 antibody may comprise a heavy chain having the amino acid sequence of SEQ ID NO: 3 and a light chain having the amino acid sequence of SEQ ID NO: 4.

In an embodiment, the anti-PD-L1 antibody may be a fully human antibody.

In an embodiment, the anti-PD-L1 antibody may retain Fc effector functions capable of stimulating antibody-dependent cellular cytotoxicity (ADCC).

In an embodiment, the anti-PD-L1 antibody may be a fully human antibody of the IgG class.

In an embodiment, the anti-PD-L1 antibody may be a fully human antibody of the IgG1 or IgG4 class.

In an embodiment, the neoadjuvant therapy is such that the anti-PD-L1 antibody may be administered intravenously or subcutaneously.

In an embodiment, the upper gastrointestinal cancer may be resectable.

In an embodiment, the upper gastrointestinal cancer may be recurrent.

In an embodiment, the upper gastrointestinal cancer may be metastatic.

In an embodiment, the upper gastrointestinal cancer may be surgically treated with curative intent.

In an embodiment, the gastric cancer may be a gastric submucosal tumor or gastric adenocarcinoma (GC).

In an embodiment, the esophageal cancer may be esophageal squamous cell carcinoma (ESCC).

In an embodiment, the liver cancer may be hepatocellular carcinoma (HCC).

In an embodiment, the neoadjuvant therapy is such that the anti-PD-L1 antibody may be administered one or more times, and each of the doses may be administered at intervals of 1 week, 2 weeks, 3 weeks, 4 weeks, 5 weeks, or 6 weeks.

In an embodiment, the neoadjuvant therapy is such that the anti-PD-L1 antibody may be administered one or more times, and each of the doses may be administered at 2-week intervals.

In an embodiment, the neoadjuvant therapy is such that the anti-PD-L1 antibody may be administered once, twice, three times, four times, five times, or six times prior to surgical resection of the tumor.

In an embodiment, the neoadjuvant therapy is such that the anti-PD-L1 antibody may be administered at a dose of 10 mg/kg to 30 mg/kg based on the patient's body weight.

In an embodiment, the neoadjuvant therapy is such that the anti-PD-L1 antibody may be administered at a dose of 20 mg/kg.

In an embodiment, surgical resection of the tumor may be performed between 11 and 84 days, or between 11 and 42 days, after the last administration of the anti-PD-L1 antibody.

According to yet another aspect of the present disclosure, there is provided a kit comprising, as neoadjuvant therapy for treating a tumor or inhibiting tumor proliferation in a patient with upper gastrointestinal cancer, a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) together with a manual for using the PD-L1 inhibitor.

In an embodiment, the manual included in the kit comprises administering a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) as neoadjuvant therapy to a patient with upper gastrointestinal cancer once, twice, three times, four times, five times, or six times at a dose of 20 mg/kg at intervals of 10 to 18 days prior to surgical resection of the tumor.

According to still yet another aspect of the present disclosure, there is provided a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) for use in a method for treating a tumor or inhibiting tumor growth, the method comprising: (a) selecting a patient with upper gastrointestinal cancer; (b) administering a therapeutically effective amount of the PD-L1 inhibitor to the patient; and (c) surgically resecting a tumor of the upper gastrointestinal cancer after step (b).

According to still yet another aspect of the present disclosure, there is provided a method for treating a tumor or inhibiting tumor growth, comprising: (a) selecting a patient with resectable gastric cancer; (b) administering a therapeutically effective amount of a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) to the patient; and (c) surgically resecting a tumor of the gastric cancer after step (b).

According to still yet another aspect of the present disclosure, there is provided a method for treating a tumor or inhibiting tumor growth, comprising: (a) selecting a patient with resectable esophageal cancer; (b) administering a therapeutically effective amount of a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) to the patient; and (c) surgically resecting a tumor of the esophageal cancer after step (b).

According to still yet another aspect of the present disclosure, there is provided a method for treating a tumor or inhibiting tumor growth, comprising: (a) selecting a patient with resectable liver cancer; (b) administering a therapeutically effective amount of a PD-L1 inhibitor (for example, an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2) to the patient; and (c) surgically resecting a tumor of the liver cancer after step (b).

**Effects of Invention**

Neoadjuvant therapy using the anti-PD-L1 antibody exhibited good tolerability and promising antitumor activity in patients with resectable, microsatellite-stable gastric cancer, esophageal cancer, and hepatocellular carcinoma. In an example of the present disclosure, administration of IMC-001 as preoperative therapy resulted in low-grade treatment-related adverse events (TRAEs); partial response (PR) or stable disease (SD) among patients with measurable lesions; and metabolic partial response (mPR), metabolic stable disease (mSD), or metabolic progressive disease (mPD) among patients evaluable for metabolic response. In addition, it was identified that IMC-001 administered as preoperative therapy resulted in varying degrees of tumor necrosis or fibrosis and led to a reduction of residual viable tumor cells to less than 50% in some patients.

**Brief Description of Drawings**

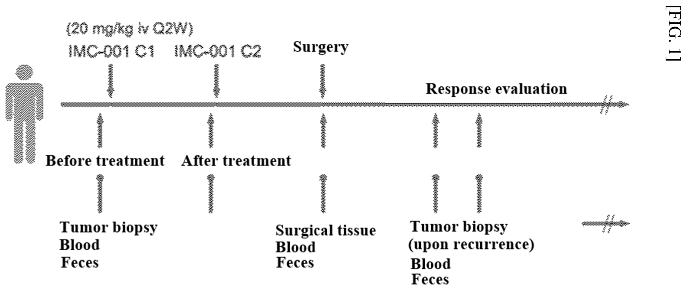

FIG. 1 schematically illustrates a procedure for a clinical trial according to an embodiment of the present disclosure.

FIG. 2 illustrates clinical tumor responses as a result of a clinical trial according to an embodiment of the present disclosure. PR indicates partial response, and SD indicates stable disease.

FIG. 3 illustrates clinical tumor responses as a result of a clinical trial according to an embodiment of the present disclosure. mPR indicates metabolic partial response, mSD indicates metabolic stable disease, and mPD indicates metabolic progressive disease.

FIG. 4 illustrates pathological responses as a result of a clinical trial according to an embodiment of the present disclosure.

## Mode for Carrying out Invention

The detailed description of the present disclosure described below will be described with reference to specific drawings (if any) regarding specific embodiments in which the present disclosure may be practiced, but the present disclosure is not limited thereto, but is limited only by the appended claims with respect to any scope identical to or equivalent to that described in the claims. It should be understood that the various embodiments/examples of the present disclosure are different from each other but do not need to be mutually exclusive. For example, certain shapes, structures, and features described herein may be altered from one embodiment/example to another, or may be realized in a combination of a plurality of embodiments/examples, without departing from the technical ideas and scope of the present disclosure. Technical and academic terms used in this specification, unless otherwise defined, have the same meanings as those commonly used in the field to which the present disclosure belongs. In case of a conflict, the specification that contains the definition shall control. The definitions of terms set forth in this specification shall apply for the purpose of interpreting this specification, and terms expressed in the singular form shall be construed to refer also to the plural form (that is, at least one) unless the context makes it inappropriate, and vice versa.

## Method for Treating Cancer or Inhibiting Cancer Proliferation

In an aspect of the present disclosure, there is provided a method for treating a tumor or inhibiting tumor proliferation, comprising selecting a patient with surgically resectable upper gastrointestinal cancer (for example, localized gastric cancer, esophageal cancer, or liver cancer), and administering to the patient a PD-L1 inhibitor (for example, the anti-PD-L1 antibody IMC-001 or a bioequivalent thereof) as neoadjuvant therapy prior to surgical resection of the tumor in the patient.

In an embodiment, the methods disclosed herein may further comprise administering the PD-L1 inhibitor (for example, the anti-PD-L1 antibody IMC-001 or a bioequivalent thereof) to the patient as adjuvant therapy after completion of surgery for treating the upper gastrointestinal cancer (for example, gastric cancer, esophageal cancer, or liver cancer).

As used herein, "gastric cancer" collectively refers to carcinomas (malignant tumors) that occur in the stomach, including gastric adenocarcinoma, lymphoma, gastric submucosal tumors, and leiomyosarcomas, and the like. Gastric cancer may occur in any part of the stomach and may spread throughout the stomach and to other organs, in particular, the esophagus, lungs, and liver. In an embodiment, the gastric cancer is gastric adenocarcinoma. In an embodiment, the gastric cancer is resectable and recurrent. In an embodiment, the gastric cancer is metastatic.

As used herein, "esophageal cancer" refers to cancer that occurs in the esophagus, which is classified by location into cervical esophageal cancer, thoracic esophageal cancer, and gastroesophageal junction cancer, and is classified by cellular morphology into squamous cell carcinoma, adenocarcinoma, sarcoma, lymphoma, melanoma, and the like. In an embodiment, the esophageal cancer is esophageal squamous cell carcinoma (ESCC). In an embodiment, the esophageal cancer is resectable and recurrent. In an embodiment, the esophageal cancer is metastatic.

As used herein, "liver cancer" refers to cancers of the liver, such as hepatocellular carcinoma, fibrolamellar carcinoma, cholangiocarcinoma, angiosarcoma, and hepatoblastoma. In an embodiment, the liver cancer is hepatocellular carcinoma (HCC). In an embodiment, the liver cancer is resectable and recurrent. In an embodiment, the liver cancer is metastatic.

In some embodiments, the patient is a surgical candidate for resecting a gastric cancer, esophageal cancer, or liver cancer tumor. In some embodiments, the patient has gastric cancer, esophageal cancer, or liver cancer for which curative surgery is intended.

As used herein, the terms "treating," "treat," and the like, mean to alleviate or reduce the severity of at least one symptom or indication, to eliminate the causation of symptoms either on a temporary or permanent basis, to delay or inhibit tumor growth, to reduce tumor cell load or tumor burden, to promote tumor regression, to cause tumor shrinkage, necrosis and/or disappearance, to prevent tumor recurrence, to prevent or inhibit metastasis, to inhibit metastatic tumor growth, to eliminate the need for surgery, and/or to increase duration of survival of the subject. In many embodiments, the terms "tumor," "lesion," "tumor lesion," "cancer," and "malignancy" are used interchangeably and refer to one or more cancerous growths.

As used herein, the term "recurrent" refers to a frequent or repeated diagnosis of gastric cancer, esophageal cancer, or liver cancer in a patient or a frequent or repeated occurrence of individual tumors, such as primary tumors and/or new tumors that may represent recurrence of a prior tumor. In certain embodiments, administration of the PD-L1 inhibitor inhibits the recurrence of gastric cancer, esophageal cancer, or liver cancer tumor in the patient.

As used herein, the expression "a subject in need of a PD-L1 inhibitor" means a human or non-human mammal that exhibits one or more symptoms or indications of gastric cancer, esophageal cancer, or liver cancer, and/or who has been diagnosed with gastric cancer, esophageal cancer, or liver cancer and who needs treatment for the same. In many embodiments, the terms "subject" and "patient" are used interchangeably. The expression includes subjects with primary, established, or recurrent tumors (advanced malignancies). In certain embodiments, the expression includes human subjects that have and/or need treatment for recurrent but non-metastatic gastric cancer, esophageal cancer, or liver cancer. In certain embodiments, the expression includes patients with a solid tumor that is resistant to or refractory to or is inadequately controlled by prior therapy (for example, surgery or treatment with another anti-cancer agent other than IMC-001 or a bioequivalent thereof). In certain embodiments, the expression includes subjects with gastric cancer, esophageal cancer, or liver cancer who are not candidates for curative surgery.

In certain embodiments, the methods of the present disclosure are used for treating a subject with a solid tumor. As used herein, the term "solid tumor" refers to an abnormal mass of tissue that usually does not contain cyst or liquid areas. Solid tumors may be benign (non cancer) or malignant (cancer). For the purposes of the present disclosure, the term "solid tumor" means malignant solid tumors. The term includes different types of solid tumors named for the cell types that form them, namely, sarcomas, carcinomas, and blastomas.

In certain embodiments, the disclosed methods comprise administering a therapeutically effective amount of a PD-L1 inhibitor (for example, the anti-PD-L1 antibody IMC-001 or a bioequivalent thereof) in combination with an additional therapeutic agent or therapy. The additional therapeutic agent or therapy may be administered for increasing anti-tumor efficacy, for reducing toxic effects of one or more therapies and/or for reducing the dosage of one or more therapies. In various embodiments, the additional therapeutic agent or therapy may include one or more of: an anti-viral therapy (for example, cidofovir), photodynamic therapy, a PD-1 inhibitor (for example, an antibody against PD-1 known in the art, such as nivolumab or pembrolizumab), a lymphocyte activation gene 3 (LAG3) inhibitor (for example, an anti-LAG3 antibody), a cytotoxic T-lymphocyte-associated protein 4 (CTLA-4) inhibitor (for example, ipilimumab), a glucocorticoid-induced tumor necrosis factor receptor (GITR) agonist (for example, anti-GITR antibody), a T-cell immunoglobulin and mucin-containing-3 (TIM3) inhibitor, a B- and T-lymphocyte attenuator (BTLA) inhibitor, a T-cell immunoreceptor with Ig and ITIM domains (TIGIT) inhibitor, a CD38 inhibitor, a CD47 inhibitor, an antagonist of another T-cell co-inhibitor or ligand (for example, an antibody to CD-28, 2B4, LY108, LAIR1, ICOS, CD160, or VISTA), a CD20 inhibitor (for example, an anti-CD20 antibody, or a bispecific CD3/CD20 antibody), an indoleamine-2,3-dioxygenase (IDO) inhibitor, a CD28 activator, a vascular endothelial growth factor (VEGF) antagonist (for example, "VEGF-Trap" such as aflibercept or other VEGF-inhibiting fusion protein as set forth in US 7087411, or an anti-VEGF antibody or antigen-binding fragment thereof (for example, bevacizumab or ranibizumab), or a small molecule kinase inhibitor of VEGF receptor (for example, sunitinib, sorafenib, pazopanib, or ramucirumab), an angiopoietin-2 (Ang2) inhibitor, a transforming growth factor beta (TGFβ) inhibitor, an epidermal growth factor receptor (EGFR) inhibitor (for example, erlotinib, cetuximab), an agonist to a co-stimulatory receptor (for example, an agonist to CD28, 4-1BB, or OX40), an antibody to a tumor-specific antigen (for example, CA9, CA125, melanoma-associated antigen 3 (MAGE3), carcinoembryonic antigen (CEA), vimentin, tumor-M2-PK, prostate-specific antigen (PSA), mucin-1, MART-1, and CA19-9), a vaccine (for example, Bacillus Calmette-Guerin or a cancer vaccine), an adjuvant to increase antigen presentation (for example, granulocyte-macrophage colony-stimulating factor), an oncolytic virus, a cytotoxin, a chemotherapeutic agent (for example, pemetrexed, dacarbazine, temozolomide, cyclophosphamide, docetaxel, doxorubicin, daunorubicin, cisplatin, carboplatin, gemcitabine, methotrexate, mitoxantrone, oxaliplatin, paclitaxel, topotecan, irinotecan, vinorelbine, and vincristine), platinum-based chemotherapy (for example, platinum-doublet chemotherapy), a tyrosine kinase inhibitor (for example, lenvatinib, regorafenib, and cabozantinib), an IL-6R inhibitor, an IL-4R inhibitor, an IL-10 inhibitor, a cytokine such as IL-2, IL-7, IL-12, IL-21, and IL-15, an antibody drug conjugate (ADC) (for example, anti-CD19-DM4 ADC, and anti-DS6-DM4 ADC), chimeric antigen receptor T cells (for example, CD19-targeted T cells), an anti-inflammatory drug such as a corticosteroid, a non-steroidal anti-inflammatory drug (NSAID), and a dietary supplement such as an antioxidant.

As used herein, the term "anti-viral therapy" refers to any agent, drug or therapy used to treat, prevent, or ameliorate a viral infection in a host subject, including but not limited to: zidovudine, lamivudine, abacavir, ribavirin, lopinavir, efavirenz, cobicistat, tenofovir, rilpivirine, analgesics, corticosteroids, and combinations thereof. In the context of the present disclosure, chronic viral infections include infections caused by viruses, including, but not limited to, human immunodeficiency virus (HIV), hepatitis B virus (HBV), and hepatitis C virus (HCV).

In certain embodiments, administration of a therapeutically effective amount of the PD-L1 inhibitor (for example, the anti-PD-L1 antibody IMC-001 or a bioequivalent thereof) to a subject suffering from gastric cancer, esophageal cancer, or liver cancer results in enhanced inhibition of tumor proliferation, for example, higher tumor regression in the treated subject.

In certain embodiments, administration of the PD-L1 inhibitor leads to one or more of: (i) improvement in tumor response rate, for example, improvement in overall response rate, complete response, or partial response, compared to a subject treated with surgical resection alone or an untreated subject; (ii) delay in tumor growth and progression (for example, tumor growth may be delayed by about 3 days, more than 3 days, about 7 days, more than 7 days, more than 15 days, more than 1 month, more than 3 months, more than 6 months, more than 1 year, more than 2 years, or more than 3 years in the treated subject, as compared to a subject treated with surgical resection alone or an untreated subject); (iii) increased disease-free survival (DFS) from date of treatment until recurrence of tumor or death, as compared to a subject treated with surgical resection alone or an untreated subject; and (iv) improved overall response rate, complete response, or partial response, as compared to a subject treated with surgical resection alone or an untreated subject. In certain embodiments, administering to a subject suffering from gastric cancer, esophageal cancer, or liver cancer a therapeutically effective amount of the PD-L1 inhibitor (for example, the anti-PD-L1 antibody IMC-001 or a bioequivalent thereof) prevents tumor recurrence and/or increases duration of survival of the subject, for example, increases duration of survival by more than 15 days, more than 1 month, more than 3 months, more than 6 months, more than 12 months, more than 18 months, more than 24 months, more than 36 months, or more than 48 months as compared to a subject treated with surgical resection alone or an untreated subject.

In certain embodiments, administering to a subject suffering from gastric cancer, esophageal cancer, or liver cancer a therapeutically effective amount of the PD-L1 inhibitor (for example, the anti-PD-L1 antibody IMC-001 or a bioequivalent thereof) leads to increased overall survival (OS) or progression-free survival (PFS) of the subject as compared to a subject treated with surgical resection alone. In certain embodiments, the PFS is increased by at least 1 month, at least 2 months, at least 3 months, at least 4 months, at least 5 months, at least 6 months, at least 7 months, at least 8 months, at least 9 months, at least 10 months, at least 11 months, at least 1 year, at least 2 years, or at least 3 years as compared to a subject treated with surgical resection alone. In certain embodiments, the OS is increased by at least 1 month, at least 2 months, at least 3 months, at least 4 months, at least 5 months, at least 6 months, at least 7 months, at least 8 months, at least 9 months, at least 10 months, at least 11 months, at least 1 year, at least 2 years, or at least 3 years as compared to a subject treated with surgical resection alone.

**PD-L1 Inhibitors**

The methods disclosed herein comprise administering a therapeutically effective amount of a PD-L1 inhibitor, wherein the PD-L1 inhibitor is an anti-PD-L1 antibody, such as the fully human anti-PD-L1 antibody IMC-001 or a bioequivalent thereof. As used herein, the term "bioequivalent" refers to anti-PD-L1 antibodies or PD-L1-binding proteins or fragments thereof that are pharmaceutical equivalents or pharmaceutical alternative whose rate and/or extent of absorption do not show a significant difference with that of the IMC-001 antibody when administered at the same molar dose under similar experimental conditions, either single dose or multiple dose. In the context of the present disclosure, the term "bioequivalent" includes antigen-binding proteins that bind to PD-L1 and do not have clinically meaningful differences from the IMC-001 antibody with respect to safety, purity, and/or potency.

The term "antibody," as used herein, is intended to refer to immunoglobulin molecules comprised of four polypeptide chains, two heavy (H) chains and two light (L) chains interconnected by disulfide bonds (i.e., "full antibody molecules"), as well as multimers thereof (for example, IgM) or antigen-binding fragments thereof. Each heavy chain is comprised of a heavy chain variable region ("HCVR" or "VH") and a heavy chain constant region (comprised of domains CH1, CH2 and CH3). Each light chain is comprised of a light chain variable region ("LCVR" or "VL") and a light chain constant region (CL). The VH and VL regions can be further subdivided into regions of hypervariability, termed complementarity determining regions (CDRs), interspersed with regions that are more conserved, termed framework regions (FRs). Each of VH and VL is composed of three CDRs and four FRs, arranged from amino-terminus to carboxy-terminus in the following order: FR1, CDR1, FR2, CDR2, FR3, CDR3, FR4. In certain embodiments, the FRs of the antibody (or antigen binding fragment thereof) may be identical to the human germline sequences or may be naturally or artificially modified. An amino acid consensus sequence may be defined based on a side-by-side analysis of two or more CDRs. The term "antibody," as used herein, also includes antigen-binding fragments of full antibody molecules.

As used herein, the terms "antigen-binding fragment" of an antibody, "antigen-binding portion" of an antibody, and the like, include any naturally occurring, enzymatically obtainable, synthetic, or genetically engineered polypeptide or glycoprotein that specifically binds an antigen to form a complex. Antigen-binding fragments of an antibody may be derived, for example, from full antibody molecules using any suitable standard techniques such as proteolytic digestion or recombinant genetic engineering techniques involving the manipulation and expression of DNA encoding antibody variable and optionally constant domains. Such DNA is known and/or is readily available from, for example, commercial sources, DNA libraries (including, for example, phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add, or delete amino acids, and the like.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (for example, an isolated complementary determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (for example, monovalent nanobodies, bivalent nanobodies, and the like), small modular immunopharmaceuticals (SMIPs), and shark variable IgNAR domains, are also encompassed within the expression "antigen-binding fragment," as used herein.

An antigen-binding fragment of an antibody will typically include at least one variable domain. The variable domain may be of any size or amino acid composition and will generally include at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and may contain a VH-VH, VH-VL, or VL-VL dimers. Alternatively, the antigen-binding fragment of an antibody may contain a monomeric VH or VL domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present disclosure include: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may consist of at least 2 (for example, 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present disclosure may include a homo-dimer or hetero-dimer (or other multimer) of any of the variable and constant domain configurations listed above in non-covalent association with one another and/or with one or more monomeric VH or VL domain (for example, by disulfide bond(s)).

The antibodies used in the methods disclosed herein may be human antibodies. As used herein, the term "human antibody" refers to antibodies having variable and constant regions derived from human germline immunoglobulin sequences. The human antibodies of the present disclosure may nevertheless include amino acid residues not encoded by human germline immunoglobulin sequences (for example, mutations introduced by random or site-specific mutagenesis *in vitro* or by somatic mutation *in vivo*), for example in the CDRs and in particular CDR3. However, the term "human antibody," as used herein, is not intended to include antibodies in which CDR sequences derived from the germline of another mammalian species, such as a mouse, have been grafted onto human framework sequences. In an embodiment, all variable and constant domains may be derived from human immunoglobulin sequences (fully human antibodies).

The antibodies used in the methods disclosed herein may be recombinant human antibodies. As used herein, the term "recombinant human antibody" includes all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (for example, a mouse) that is transgenic for human immunoglobulin genes (see, for example, Taylor et al., (1992) Nucl. Acids Res. 20:6287-6295), or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo*.

In some embodiments, the PD-L1 inhibitor is an anti-PD-L1 antibody (for example, IMC-001) that comprises three heavy chain complementarity-determining regions (HCDRs) of a heavy chain variable region (HCVR) comprising or consisting of the amino acid sequence of SEQ ID NO: 1, and three light chain complementarity-determining regions (LCDRs) of a light chain variable region (LCVR) comprising or consisting of the amino acid sequence of SEQ ID NO: 2. In some embodiments, the anti-PD-L1 antibody comprises three HCDRs (HCDR1, HCDR2, and HCDR3) and three LCDRs (LCDR1, LCDR2, and LCDR3), wherein HCDR1 comprises or consists of the amino acid sequence of SEQ ID NO: 5; HCDR2 comprises or consists of the amino acid sequence of SEQ ID NO: 6; HCDR3 comprises or consists of the amino acid sequence of SEQ ID NO: 7; LCDR1 comprises or consists of the amino acid sequence of SEQ ID NO: 8; LCDR2 comprises or consists of the amino acid sequence of SEQ ID NO: 9; and LCDR3 comprises or consists of the amino acid sequence of SEQ ID NO: 10. In certain embodiments, the anti-PD-L1 antibody comprises an HCVR comprising or consisting of SEQ ID NO: 1, and an LCVR comprising or consisting of SEQ ID NO: 2. In certain embodiments, the anti-PD-L1 antibody comprises a heavy chain and a light chain, wherein the heavy chain may comprise or consist of the amino acid sequence of SEQ ID NO: 3 and the light chain may comprise or consist of the amino acid sequence of SEQ ID NO: 4. In this specification, a fully human IgG1 monoclonal antibody, which comprises a heavy chain having the amino acid sequence of SEQ ID NO: 3 and a light chain having the amino acid sequence of SEQ ID NO: 4, is referred to as "IMC-001."

In some embodiments, the anti-PD-L1 antibody may comprise an HCVR having at least 90%, at least 95%, at least 97%, or at least 98% sequence identity to SEQ ID NO: 1. In some embodiments, the anti-PD-L1 antibody may comprise an LCVR having at least 90%, at least 95%, at least 97%, or at least 98% sequence identity to SEQ ID NO: 2. In some embodiments, the anti-PD-L1 antibody may comprise an HCVR having at least 90%, at least 95%, at least 97%, at least 98% sequence identity to SEQ ID NO: 1 and an LCVR having at least 90%, at least 95%, at least 97%, at least 98% sequence identity to SEQ ID NO: 2. Sequence identity may be measured by methods known in the art (for example, GAP, BESTFIT, and BLAST).

In some embodiments, the sequence identity may be determined with respect to regions excluding CDRs (for example, framework sequences). In some embodiments, the bioequivalent of the IMC-001 antibody is an anti-PD-L1 antibody comprising an HCVR having at least 90%, at least 95%, at least 97%, or at least 98% sequence identity to the regions excluding HCDR1, HCDR2, and HCDR3 in SEQ ID NO: 1. In some embodiments, the bioequivalent of the IMC-001 antibody is an anti-PD-L1 antibody comprising an LCVR having at least 90%, at least 95%, at least 97%, or at least 98% sequence identity to the regions excluding LCDR1, LCDR2, and LCDR3 in SEQ ID NO: 2. In some embodiments, the bioequivalent of IMC-001 is an anti-PD-L1 antibody comprising an HCVR having at least 90%, at least 95%, at least 97%, or at least 98% sequence identity to the regions excluding HCDR1, HCDR2, and HCDR3 in SEQ ID NO: 1, and an LCVR having at least 90%, at least 95%, at least 97%, or at least 98% sequence identity to the regions excluding LCDR1, LCDR2, and LCDR3 in SEQ ID NO: 2. In some embodiments, the bioequivalent of the IMC-001 antibody is an anti-PD-L1 antibody that comprises an LCVR having at least 90%, at least 95%, at least 97%, at least 98% sequence identity to the regions excluding LCDR1, LCDR2, and LCDR3 in SEQ ID NO: 2 and has the same amino acids at positions 3 and 5, from the N-terminus to the C-terminus, as those at positions 3 and 5 of SEQ ID NO: 2.

In some embodiments, the IMC-001 antibodies or bioequivalents thereof may be produced, in particular, in CHO cells without involving light chain fragmentation, by having the above-described light chain amino acid sequence.

In some embodiments, the bioequivalent of the IMC-001 antibody is an anti-PD-L1 antibody that comprises an HCVR comprising the amino acid sequence of SEQ ID NO: 1 having no more than five amino acid substitutions. In some embodiments, the bioequivalent of IMC-001 is an anti-PD-L1 antibody that comprises an LCVR comprising the amino acid sequence of SEQ ID NO: 2 having no more than two amino acid substitutions. In some embodiments, the bioequivalent of IMC-001 is an anti-PD-L1 antibody that comprises an HCVR comprising the amino acid sequence of SEQ ID NO: 1 having no more than five amino acid substitutions, and an LCVR comprising the amino acid sequence of SEQ ID NO: 2 having no more than two amino acid substitutions.

The antibody used in the methods disclosed herein may be a humanized antibody. As used herein, the term "humanized antibody" has a sequence that differs from that of an antibody derived from a non-human species by one or more amino acid substitutions, deletions, and/or additions, such that the humanized antibody is less likely to induce an immune response and/or induces a less severe immune response, as compared to the non-human species antibody, when it is administered to a human subject. In an embodiment, certain amino acids within the framework and constant domains of the heavy and/or light chains of the non-human species antibody are mutated to generate the humanized antibody. In another embodiment, the constant domain(s) of a human antibody are fused to the variable domain(s) of a non-human species. In another embodiment, one or more amino acid residues in one or more CDR sequences of a non-human antibody may be altered to reduce the likely immunogenicity of the non-human antibody when it is administered to a human subject, wherein the altered amino acid residues either are not critical for immunospecific binding of the antibody to its antigen, or the alterations made to the amino acid sequence are conservative changes such that the binding of the humanized antibody to the antigen is not significantly worse than the binding of the non-human antibody to the antigen. Examples of how to make humanized antibodies may be found in U.S. Patent Nos. 6,054,297, 5,886,152, and 5,877,293.

In an embodiment, the anti-PD-L1 antibody is a fully human antibody of the IgG class that binds to a PD-L1 epitope with an affinity of $10^{-6}$ M or less. In an embodiment, the anti-PD-L1 antibody may be either of the IgG1 or IgG4 subclass. In an embodiment, there is provided a fully human antibody of the IgG class (for example, IgG1 or IgG4) that binds to a PD-L1 epitope, wherein the antibody comprises a heavy chain variable domain comprising an amino acid sequence that is at least 90% identical to the amino acid sequence set forth in SEQ ID NO: 1, and a light chain variable domain comprising an amino acid sequence that is at least 90% identical to the amino acid sequence set forth in SEQ ID NO: 2. In an embodiment, there is provided a fully human antibody of the IgG class (for example, IgG1 or IgG4) that binds to a PD-L1 epitope, wherein the antibody comprises a heavy chain variable domain that comprises CDR1, CDR2, and CDR3 domains having the amino acid sequences of SEQ ID NOs: 5, 6, and 7, respectively, and a light chain variable domain that comprises CDR1, CDR2, and CDR3 domains having the amino acid sequences of SEQ ID NOs: 8, 9, and 10, respectively.

More specific details regarding the anti-PD-L1 antibodies of the present disclosure can be found in International Publication WO 2017/132562 A1. The IMC-001 antibody comprises a heavy chain variable domain region sequence of SEQ ID NO: 1 and a light chain variable domain region sequence of SEQ ID NO: 2 as disclosed in the document, the entire disclosure of which is incorporated herein by reference.

## Preparation of PD-L1 Inhibitors

Antigen-binding proteins may be prepared by any of a number of conventional techniques. In an embodiment of the present disclosure, there is provided a monoclonal antibody that binds to PD-L1. For example, monoclonal antibodies may be purified from cells that naturally express them (for example, an antibody can be purified from a hybridoma that produces it) or produced in recombinant expression systems, using any technique known in the art. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, N.Y., (1988).

Monoclonal antibodies may be produced using any technique known in the art, for example, by immortalizing spleen cells collected from a transgenic animal after completion of an immunization schedule. The spleen cells may be immortalized using any technique known in the art, for example, by fusing them with myeloma cells to produce hybridomas. Myeloma cells for use in hybridoma-producing fusion procedures preferably are non-antibody-producing, have high fusion efficiency, and have enzyme deficiencies that render them incapable of growing in certain selective media which support the growth of only the desired fusion cells (hybridomas). Examples of suitable cell lines for use in mouse fusions include Sp-20, P3-X63/Ag8, P3-X63-Ag8.653, NS1/1.Ag 41, Sp210-Ag14, FO, NSO/U, MPC-11, MPC11-X45-GTG 1.7, and S194/5XX0 Bul; examples of cell lines used in rat fusions include R210.RCY3, Y3-Ag 1.2.3, IR983F, and 4B210. Other cell lines useful for cell fusions include U-266, GM1500-GRG2, LICR-LON-HMy2, and UC729-6.

In an example, polypeptides are produced by recombinant DNA methods by inserting a nucleic acid sequence (for example, cDNA) encoding the polypeptide into a recombinant expression vector and expressing the DNA sequence under conditions that promote expression.

For recombinant production of an anti-PD-L1 antibody, a nucleic acid encoding an antibody is isolated and inserted into one or more vectors for further cloning and/or expression in a host cell. Such nucleic acid may be readily isolated and sequenced using conventional procedures (for example, using oligonucleotide probes that are capable of binding specifically to genes encoding the heavy and light chains of the antibody).

Nucleic acids encoding anti-PD-L1 antibodies (or fragments thereof) disclosed herein may be chemically synthesized. Codon usage may be selected to improve expression in a cell. Such codon usage will depend on the selected cell type. Specialized codon usage patterns have been developed for *E. coli* and other bacteria, as well as for mammalian cells, plant cells, yeast cells, and insect cells. See, for example, Mayfield et al., Proc. Natl. Acad. Sci. USA. 2003 100(2):438-42; Sinclair et al., Protein Expr. Purif. 2002 (1):96-105; Connell N D. Curr. Opin. Biotechnol. 2001 12(5):446-9; Makrides et al., Microbiol. Rev. 1996 60(3):512-38; and Sharp et al., Yeast. 1991 7(7):657-78.

General techniques for nucleic acid manipulation are described, for example, in Sambrook et al., Molecular Cloning: A Laboratory Manual, Vols. 1-3, Cold Spring Harbor Laboratory Press, 2 ed., 1989, or F. Ausubel et al., Current Protocols in Molecular Biology (Green Publishing and Wiley-Interscience: New York, 1987) and periodic updates, herein incorporated by reference. The DNA encoding the polypeptide is operably linked to suitable transcriptional or translational regulatory elements derived from mammalian, viral, or insect genes. Such regulatory elements include a transcriptional promoter, an optional operator sequence to control transcription, a sequence encoding suitable mRNA ribosomal binding sites, and sequences that control the termination of transcription and translation. The ability to replicate in a host, usually conferred by an origin of replication, and a selection gene to facilitate recognition of transformants are additionally incorporated.

The recombinant DNA may also include any type of protein tag sequence that can be useful for purifying the protein. Examples of protein tags include, but are not limited to, a histidine tag, a FLAG tag, a myc tag, an HA tag, or a GST tag. Appropriate cloning and expression vectors for use with bacterial, fungal, yeast, and mammalian cellular hosts can be found in Cloning Vectors: A Laboratory Manual, (Elsevier, N.Y., 1985).

The expression construct is introduced into the host cell using a method appropriate for the host cell. Various methods for introducing nucleic acids into host cells are known in the art, including, but not limited to, electroporation; transfection employing calcium chloride, rubidium chloride, calcium phosphate, DEAE-dextran, or other substances; microprojectile bombardment; lipofection; and infection (where the vector is an infectious agent). Suitable host cells include prokaryotes, yeast, mammalian cells, or bacterial cells, as described in more detail below.

Any expression system known in the art can be used to make the recombinant polypeptides (for example, recombinant antibodies) of the present disclosure. In general, host cells are transformed with a recombinant expression vector that comprises DNA encoding a desired polypeptide. Among the host cells that may be employed are prokaryotes, yeast, or higher eukaryotic cells. Prokaryotes include gram-negative or gram-positive organisms, for example, *E. coli* or bacilli. Higher eukaryotic cells include insect cells and established cell lines of mammalian origin. Suitable host cells for cloning or expression of antibody-encoding vectors include prokaryotic or eukaryotic cells described herein. For example, antibodies may be produced in bacteria, in particular when glycosylation and Fc effector function are not required. For expression of antibody fragments and polypeptides in bacteria, see, for example, U.S. Patent Nos. 5,648,237, 5,789,199, and 5,840,523. (See also Charlton, Methods in Molecular Biology, Vol. 248 (B. K. C. Lo, ed., Humana Press, Totowa, N.J., (2003), pp. 245-254), which describes expression of antibody fragments in *E. coli*.) After expression, the antibody may be isolated from the bacterial cell paste in a soluble fraction and may be further purified. Higher eukaryotic cells include insect cells and established cell lines of mammalian origin. Examples of suitable mammalian host cell lines include the COS-7 cell line of monkey kidney cells (ATCC CRL 1651) (Gluzman et al., 1981, Cell 23:175), L cells, 293 cells, C127 cells, 3T3 cells (ATCC CCL 163), Chinese hamster ovary (CHO) cells, HeLa cells, BHK (ATCC CRL 10) cell lines, and the CV1/EBNA cell line derived from the African green monkey kidney cell line CV1 (ATCC CCL 70) as described by McMahan et al., 1991, EMBO J. 10: 2821. Suitable cloning and expression vectors for use with bacterial, fungal, yeast, and mammalian cellular hosts are described by Pouwels et al. (Cloning Vectors: A Laboratory Manual, (Elsevier, N.Y., 1985). CV1/EBNA cell line derived from the African green monkey kidney cell line CV1 (ATCC CCL 70) as described by McMahan et al., 1991, EMBO J. 10: 2821. Appropriate cloning and expression vectors for use with bacterial, fungal, yeast, and mammalian cellular hosts are described by Pouwels et al. (Cloning Vectors: A Laboratory Manual, Elsevier, N.Y., 1985).

In certain embodiments, vertebrate cells may be used as hosts to express anti-PD-L1 antibodies or fragments thereof. For example, mammalian cell lines that are adapted to grow in suspension may be useful. Other examples of useful mammalian host cell lines include monkey kidney CV1 line transformed by SV40 (COS-7); human embryonic kidney line (293 or 293 cells as described, for example, in Graham et al., J. Gen Virol. 36:59 (1977)); baby hamster kidney cells (BHK); mouse sertoli cells (TM4 cells as described, for example, in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (HepG2); mouse mammary tumor (MMT 060562); TRI cells, as described, for example, in Mather et al., Annals N.Y. Acad. Sci. 383:44-68 (1982); MRC 5 cells; and FS4 cells. Other useful mammalian host cell lines include Chinese hamster ovary (CHO) cells, including DHFR-CHO cells (Urlaub et al., Proc. Natl. Acad. Sci. USA 77:4216 (1980)); and myeloma cell lines, such as Y0, NS0, and Sp2/0. For a review of certain mammalian host cell lines suitable for antibody production, see, for example, Yazaki and Wu, Methods in Molecular Biology, Vol. 248 (B. K. C. Lo, ed., Humana Press, Totowa, N.J.), pp. 255-268 (2003). Examples of suitable mammalian host cell lines include the COS-7 cell line of monkey kidney cells (ATCC CRL 1651) (Gluzman et al., 1981, Cell 23:175), L cells, 293 cells, C127 cells, 3T3 cells (ATCC CCL 163), Chinese hamster ovary (CHO) cells, HeLa cells, BHK (ATCC CRL 10) cell lines, and the CV1/EBNA cell line derived from the African green monkey kidney cell line CV1 (ATCC CCL 70) as described by McMahan et al., 1991, EMBO J. 10: 2821.

In addition to prokaryotes, eukaryotic microbes, such as filamentous fungi or yeasts, are suitable cloning or expression hosts for antibody-encoding vectors, including fungi and yeast strains whose glycosylation pathways have been humanized, resulting in production of an antibody with a partially or fully human glycosylation pattern. See, Gerngross, Nat. Biotech. 22:1409-1414 (2004), and Li et al., 24:210-215 (2006).

Suitable host cells for the expression of glycosylated antibodies are also derived from multicellular organisms (invertebrates and vertebrates). Examples of invertebrate cells include plant and insect cells. Various baculoviral strains have been identified, which may be used in conjunction with insect cells, particularly for transfection of *Spodoptera frugiperda* cells.

Suitable cloning and expression vectors for use with bacterial, fungal, yeast, and mammalian cellular hosts are described by Pouwels et al. (Cloning Vectors: A Laboratory Manual, (Elsevier, N.Y., 1985).

The transformed cells can be cultured under conditions that promote expression of the polypeptide, and the polypeptide is recovered by conventional protein purification procedures. One such purification procedure includes, for example, use of affinity chromatography on a matrix having all or a portion (for example, the extracellular domain) of PD-L1 bound thereto. Polypeptides contemplated for use herein include substantially homogeneous recombinant mammalian anti-PD-L1 antibody polypeptides that are substantially free of contaminating endogenous materials.

Accordingly, antibodies may be produced using recombinant methods and compositions, for example, as described in U.S. Patent No. 4,816,567. In an embodiment, an isolated nucleic acid encoding the anti-PD-L1 antibody described herein is provided. Such nucleic acid may encode an amino acid sequence comprising the VL of the antibody and/or an amino acid sequence comprising the VH of the antibody (for example, the light and/or heavy chains of the antibody). In a further embodiment, there are provided one or more vectors (for example, expression vectors) comprising such nucleic acid. In a further embodiment, there is provided a host cell comprising such nucleic acid. In one such embodiment, a host cell comprises (for example, has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In an embodiment, the host cell is eukaryotic, for example, a Chinese hamster ovary (CHO) cell or lymphoid cell (for example, Y0, NS0, Sp20 cell). In an embodiment, there is provided a method for producing an anti-PD-L1 antibody, wherein the method comprises culturing a host cell comprising a nucleic acid encoding the antibody, as provided above, under conditions suitable for expression of the antibody, and optionally recovering the antibody from the host cell (or host cell culture medium).

The proteins disclosed herein may also be produced using cell-translation systems. For this purpose, the nucleic acid encoding the polypeptide must be modified to allow in vitro transcription to produce mRNA and to allow cell-free translation of the mRNA in the specific cell-free system being utilized (eukaryotic such as a mammalian or yeast cell-free translation system or prokaryotic such as a bacterial cell-free translation system).

Additionally, PD-L1-binding polypeptides can also be produced by chemical synthesis (for example, by the methods described in Solid Phase Peptide Synthesis, 2nd ed., 1984, The Pierce Chemical Co., Rockford, III.). Modifications to the protein can also be achieved by chemical synthesis.

The polypeptides of the present disclosure can be purified by isolation/purification methods for proteins generally known in the field of protein chemistry. Non-limiting examples thereof include extraction, recrystallization, salting out (for example, with ammonium sulfate or sodium sulfate), centrifugation, dialysis, ultrafiltration, adsorption chromatography, ion exchange chromatography, hydrophobic chromatography, normal phase chromatography, reverse phase chromatography, gel filtration, gel permeation chromatography, affinity chromatography, electrophoresis, countercurrent distribution, or any combination thereof. After purification, polypeptides may be exchanged into different buffers and/or concentrated by any of a variety of methods known in the art, including, but not limited to, filtration and dialysis.

The purified polypeptide is preferably at least 85% pure, more preferably at least 95% pure, and most preferably at least 98% pure. Regardless of the exact numerical value of the purity, the polypeptide is sufficiently pure for use as a pharmaceutical product. Antigen-binding proteins (for example, antibodies, antibody fragments, antibody derivatives, antibody muteins, and antibody variants) are polypeptides that bind to PD-L1 (preferably, human PD-L1). Antigen-binding proteins include those that inhibit biological activity of PD-L1.

Antigen-binding proteins may be prepared and screened for desired properties, by any of a number of known techniques. Certain of the techniques involve isolating a nucleic acid encoding a polypeptide chain (or portion thereof) of an antigen-binding protein of interest (for example, an anti-PD-L1 antibody), and manipulating the nucleic acid through recombinant DNA technology. The nucleic acid may be fused to another nucleic acid of interest, or altered (for example, by mutagenesis or other conventional techniques) to add, delete, or substitute one or more amino acid residues, for example.

Single-chain antibodies may be formed by linking heavy and light chain variable domain (Fv region) fragments via an amino acid bridge (short peptide linker), resulting in a single polypeptide chain. Such single-chain Fvs (scFvs) have been prepared by fusing DNA encoding a peptide linker between DNAs encoding the two variable domain polypeptides (VL and VH). The resulting polypeptides can fold back on themselves to form antigen-binding monomers, or they can form multimers (for example, dimers, trimers, or tetramers), depending on the length of a flexible linker between the two variable domains (Kortt et al., 1997, Prot. Eng. 10:423; Kortt et al., 2001, Biomol. Eng. 18:95-108). By combining different VL and VH-comprising polypeptides, one can form multimeric scFvs that bind to different epitopes (Kriangkum et al., 2001, Biomol. Eng. 18:31-40). Techniques developed for the production of single-chain antibodies include those described in U.S. Patent No. 4,946,778; Bird, 1988, Science 242:423; Huston et al., 1988, Proc. Natl. Acad. Sci. USA 85:5879; Ward et al., 1989, Nature 334:544, de Graaf et al., 2002, Methods Mol. Biol. 178:379-87.

Techniques are known for deriving an antibody of a different subclass or isotype from an antibody of interest, that is, subclass switching. Thus, IgG antibodies may be derived from an IgM antibody, for example, and vice versa. Such techniques allow the preparation of new antibodies that possess the antigen-binding properties of a given antibody (the parent antibody), but also exhibit biological properties associated with an antibody isotype or subclass different from that of the parent antibody. Recombinant DNA techniques may be employed. Cloned DNA encoding particular antibody polypeptides may be employed in such procedures, for example, DNA encoding the constant domain of an antibody of the desired isotype (Lantto et al., 2002, Methods Mol. Biol. 178:303-16). Moreover, if an IgG4 is desired, it may also be desired to introduce a point mutation (CPSCP → CPPCP) in the hinge region (Bloom et al., 1997, Protein Science 6:407) to alleviate a tendency to form inter-H-chain disulfide bonds that can lead to heterogeneity in the IgG4 antibodies.

**Pharmaceutical Compositions and Administration**

According to still yet another aspect of the present disclosure, there is provided a pharmaceutical composition comprising a PD-L1 inhibitor as neoadjuvant therapy, as disclosed herein. Such pharmaceutical compositions may be formulated with suitable pharmaceutically acceptable carriers, excipients, buffers, and other agents that provide suitable transfer, delivery, tolerance, and the like. A multitude of appropriate formulations can be found in formulary known to all pharmaceutical chemists: Remington's Pharmaceutical Sciences, Mack Publishing Company, Easton, PA. These formulations include, for example, acids, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsion carbowax (polyethylene glycols of various molecular weights), semi-solid gels, and semi-solid mixtures containing carbowax. See also Powell et al., "Compendium of excipients for parenteral formulations" PDA, J Pharm Sci Technol 52:238-311 (1998).

The dose of a PD-L1 inhibitor (for example, anti-PD-L1 antibody) may vary depending upon the age and size of a subject to be administered, target disease, conditions, route of administration, and the like. When a PD-L1 inhibitor of the present disclosure is used for treating or inhibiting the growth of gastric cancer, esophageal cancer, or liver cancer, the PD-L1 inhibitor may be administered in single or multiple doses of about 0.1 to about 100 mg/kg body weight. Depending on the severity of the condition, the frequency and duration of the treatment can be adjusted. In some embodiments, the PD-L1 inhibitor of the present disclosure may be administered at an initial dose of at least about 0.1 mg to about 800 mg, about 1 to about 1000 mg, about 1 to about 800 mg, about 5 to about 500 mg, or about 10 to about 400 mg. In some embodiments, the initial dose may be followed by administration of a second or a plurality of subsequent doses of the PD-L1 inhibitor in an amount that can be approximately the same or less than that of the initial dose, wherein the subsequent doses are given at intervals of at least 1 day to 3 days; at least 1 week; at least 2 weeks; at least 3 weeks; at least 4 weeks; at least 5 weeks; at least 6 weeks; at least 7 weeks; at least 8 weeks; at least 9 weeks; at least 10 weeks; at least 12 weeks; or at least 14 weeks. In some embodiments, the subsequent doses of the PD-L1 inhibitor following the initial dose are preferably given at intervals of at least 1 week, at least 2 weeks, at least 3 weeks, or at least 4 weeks.

Various delivery systems are known and can be used to administer the pharmaceutical composition of the present disclosure, for example, encapsulation in liposomes, microparticles, microcapsules, recombinant cells capable of expressing the mutant viruses, receptor-mediated endocytosis (see, for example, Wu et al., (1987) J. Biol. Chem. 262:4429-4432). Methods of introduction include, but are not limited to, intradermal, transdermal, intramuscular, intravenous, subcutaneous, intranasal, epidural and oral routes. The composition may be administered by any convenient route, for example by infusion or bolus injection, by absorption through epithelial or mucocutaneous linings (for example, oral mucosa, rectal and intestinal mucosa, and the like) and may be administered together with other biologically active agents. The pharmaceutical composition can be also delivered in a vesicles, in particular a liposome (see, for example, Langer (1990) Science 249:1527-1533).

The use of nanoparticles to deliver PD-L1 inhibitors of the present disclosure is also contemplated herein. Antibody-conjugated nanoparticles may be used both for therapeutic and diagnostic applications. Antibody-conjugated nanoparticles and methods of preparation and use are described in detail by Arruebo, M., et al., 2009, "Antibody-conjugated nanoparticles for biomedical applications," J. Nanomat., Vol. 2009, Article ID 439389, 24 pages. Nanoparticles may be developed and conjugated to antibodies contained in pharmaceutical compositions to target cells. Nanoparticles for drug delivery have also been described in, for example, US 8257740 or US 8246995.

In some situations, the pharmaceutical composition can be delivered in a controlled release system. In an embodiment, a pump may be used. In another embodiment, polymeric materials can be used. In yet another embodiment, a controlled release system can be placed in proximity of the composition's target, thus requiring only a fraction of the systemic dose.

Injectable preparations may include dosage forms for intravenous, subcutaneous, intracranial, and intramuscular injections, drip infusions, and the like. These injectable preparations may be prepared by methods publicly known. In an embodiment, the injectable preparations may be a histidine-based solution.

A pharmaceutical composition of the present disclosure can be delivered subcutaneously or intravenously with a standard needle and syringe. In addition, with respect to subcutaneous delivery, a pen delivery device readily has applications in delivering a pharmaceutical composition of the present disclosure. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Advantageously, the pharmaceutical compositions for oral or parenteral use described above are prepared in dosage forms in a unit dose suited to fit a dose of the active ingredients. Such dosage forms in a unit dose include, for example, tablets, pills, capsules, injections (ampoules), suppositories, and the like. The amount of the antibody contained is generally about 5 to about 1000 mg per dosage form in a unit dose, such as about 5 to about 600 mg, about 5 to about 350 mg, or about 10 to about 300 mg.

In certain embodiments, the present disclosure provides a pharmaceutical composition or formulation comprising a therapeutically effective amount of a PD-L1 inhibitor (for example, IMC-001 or a bioequivalent thereof) and a pharmaceutically acceptable carrier. Non-limiting examples of pharmaceutical compositions comprising an anti-PD-L1 antibody provided herein that can be used in the context of the present disclosure are disclosed in US 2019/0040137.

In addition, according to still yet another aspect of the present disclosure, there is provided a kit comprising a PD-L1 inhibitor (for example, IMC-001 or a bioequivalent thereof) for therapeutic uses as described herein. Kits typically comprise a label indicating the intended use of the contents of the kit and a manual for use. As used herein, the term "label" comprises any writing, or recorded material supplied on, in, or with the kit, or which otherwise accompanies the kit. In an embodiment, there is provided a kit for treating a patient suffering from upper gastrointestinal cancer (for example, gastric cancer, esophageal cancer, or liver cancer), the kit comprising: (a) a therapeutically effective amount of a PD-L1 inhibitor (for example, IMC-001 or a bioequivalent thereof); and (b) a manual for using the PD-L1 inhibitor in any of the methods disclosed herein.

## Dosing Regimen and Dosage

In certain embodiments, the methods of the present disclosure may comprise administering to a tumor of a subject a therapeutically effective amount of a PD-L1 inhibitor (for example, IMC-001 antibody or a bioequivalent thereof) at least one time, for example, once, twice, three times, four times, five times, six times, seven times, eight times, nine times, or ten times. For example, the therapeutic dosing regimen may comprise administering one or more doses of a PD-L1 inhibitor to the subject at intervals of about 1 day, about 2 days, about 3 days, about 4 days, about 5 days, about 6 days, about 1 week, about 2 weeks, about 3 weeks, about 4 weeks, about 5 weeks, about 6 weeks, about 7 weeks, about 8 weeks, about 12 weeks, about 1 month, about 2 months, about 3 months, or about 4 months, or at longer intervals, or as needed as long as a therapeutic response is achieved.

In certain embodiments, the PD-L1 inhibitor is administered in at least one treatment cycle, such as one, two, three, four, five, six, seven, eight, nine, or ten treatment cycles. A method according to this aspect comprises administering one or more cycles of neoadjuvant therapy to a subject in need thereof, optionally followed by one or more cycles of adjuvant therapy. Each treatment cycle comprises administering a PD-L1 inhibitor (for example, IMC-001 or a bioequivalent thereof) once, twice, three times, four times, five times, six times, seven times, eight times, nine times, ten times, or more times.

In an embodiment, the neoadjuvant therapy is such that the PD-L1 inhibitor is administered once, twice, three times, four times, five times, or six times prior to the surgical resection of the tumor. In an embodiment, the neoadjuvant therapy is such that the PD-L1 inhibitor is administered 2 to 6 times prior to the surgical resection of the tumor. In an embodiment, the neoadjuvant therapy is such that the PD-L1 inhibitor is administered for a total of two doses (two treatment cycles) prior to the surgical resection of the tumor. In an embodiment, the neoadjuvant therapy is such that the PD-L1 inhibitor is administered at least twice (two treatment cycles) and up to six times (six treatment cycles) prior to the surgical resection of the tumor. In an embodiment, the neoadjuvant therapy is such that the PD-L1 inhibitor is administered once every about 1 week, every about 2 weeks, or every about 3 weeks. In an embodiment, the neoadjuvant therapy is such that the PD-L1 inhibitor is administered at intervals of 10 to 18 days, preferably at intervals of 14 days.

In certain embodiments, each of the doses of the PD-L1 inhibitor comprises 0.1, 1, 0.3, 3, 4, 5, 6, 7, 8, 9, 10, 11, 12, 13, 14, 15, 16, 17, 18, 19, 20, 21, 22, 23, 24, 25, 26, 27, 28, 29, or 30 mg/kg of the patient's body weight. In certain embodiments, each of the doses of the PD-L1 inhibitor is 20 mg/kg. In some embodiments, the neoadjuvant therapy is such that the PD-L1 inhibitor is administered a total of two times at a dose of 20 mg/kg at intervals of 10 to 18 days prior to the surgical resection of the tumor (two cycles of administration).

The amount of a PD-L1 inhibitor (for example, IMC-001 or a bioequivalent thereof) administered to a subject according to the methods disclosed herein is generally a therapeutically effective amount. As used herein, the term "therapeutically effective amount" means an amount of a PD-L1 inhibitor administered as neoadjuvant therapy prior to performing planned surgery for the treatment of gastric cancer, esophageal cancer, or liver cancer that results in at least one of the following: (a) inhibition of tumor proliferation or an increase in tumor necrosis, tumor shrinkage, and/or tumor disappearance; (b) reduction in severity or duration of a symptom or an indication of cancer, for example, a tumor lesion; (c) delay in tumor proliferation and development; (d) inhibition of tumor metastasis; (e) prevention of recurrence of tumor proliferation; and/or (f) increase in survival of a subject with cancer, each as compared to subjects treated with surgical resection alone or non-treated subjects.

In certain embodiments, a therapeutically effective amount of the PD-L1 inhibitor (for example, IMC-001 or a bioequivalent thereof) can be from about 0.05 mg to about 1000 mg, from about 1 mg to about 800 mg, from about 5 mg to about 600 mg, from about 10 mg to about 550 mg, from about 50 mg to about 400 mg, from about 75 mg to about 350 mg, or from about 100 mg to about 300 mg of the antibody. For example, in various embodiments, the amount of the PD-L1 inhibitor is about 0.05 mg, about 0.1 mg, about 1.0 mg, about 1.5 mg, about 2.0 mg, about 5 mg, about 10 mg, about 15 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 710 mg, about 720 mg, about 730 mg, about 740 mg, about 750 mg, about 760 mg, about 770 mg, about 780 mg, about 790 mg, about 800 mg, about 810 mg, about 820 mg, about 830 mg, about 840 mg, about 850 mg, about 860 mg, about 870 mg, about 880 mg, about 890 mg, about 900 mg, about 910 mg, about 920 mg, about 930 mg, about 940 mg, about 950 mg, about 960 mg, about 970 mg, about 980 mg, about 990 mg, or about 1000 mg.

The amount of a PD-L1 inhibitor (for example, IMC-001 or a bioequivalent thereof) contained within an individual dose may be expressed in terms of milligrams of antibody per kilogram of subject's body weight (that is, mg/kg). In certain embodiments, the PD-L1 inhibitor used in the methods disclosed herein may be administered to a subject at a dose of about 0.0001 to about 100 mg/kg of a subject's body weight. In certain embodiments, an anti-PD-L1 antibody may be administered to a subject at dose of about 0.1 mg/kg to about 30 mg/kg of a subject's body weight. In certain embodiments, the methods of the present disclosure comprise administration of a PD-L1 inhibitor (for example, the anti-PD-L1 antibody IMC-001 or a bioequivalent thereof) at a dose of about 1 mg/kg to 30 mg/kg, 5 mg/kg to 30 mg/kg, 10 mg/kg to 30 mg/kg, 15 mg/kg, or 20 mg/kg of a subject's body weight.

In certain embodiments, an individual dose amount of a PD-L1 inhibitor (for example, IMC-001 or a bioequivalent thereof) administered to a patient may be less than a therapeutically effective amount, that is, a subtherapeutic dose. For example, if the therapeutically effective amount of a PD-L1 inhibitor includes 20 mg/kg, a subtherapeutic dose includes an amount less than 20 mg/kg, for example, 18 mg/kg, 16 mg/kg, 14 mg/kg, 12 mg/kg, or 10 mg/kg. As defined herein, a "subtherapeutic dose" refers to an amount of the PD-L1 inhibitor that does not lead to a therapeutic effect by itself. However, in certain embodiments, multiple subtherapeutic doses of a PD-L1 inhibitor are administered to collectively achieve a therapeutic effect in the subject.

In certain embodiments, each of the doses comprises 0.1 to 30 mg/kg (for example, 5 mg/kg, 10 mg/kg, 15 mg/kg, 20 mg/kg, 20 mg/kg, or 25 mg/kg) of the PD-L1 inhibitor (for example, IMC-001 or a bioequivalent thereof) based on a patient's body weight. In some other embodiments, each of the doses comprises from 5 to 600 mg of the PD-L1 inhibitor, for example, 5 mg, 10 mg, 15 mg, 20 mg, 25 mg, 30 mg, 40 mg, 45 mg, 50 mg, 100 mg, 150 mg, 200 mg, 250 mg, 300 mg, 400 mg, 500 mg, or 600 mg of the PD-L1 inhibitor.

In an embodiment, a therapeutically effective amount of the PD-L1 inhibitor (for example, IMC-001 or a bioequivalent thereof) is 20 mg/kg administered intravenously as neoadjuvant therapy prior to performing a planned surgery for gastric cancer, esophageal cancer, or liver cancer. In some embodiments, another therapeutically effective amount of the PD-L1 inhibitor (for example, IMC-001 or a bioequivalent thereof) is 20 mg/kg administered intravenously as adjuvant therapy after surgery.

In certain embodiments, surgical resection of the tumor is performed after 11 days or within 84 days from the last administration of the PD-L1 inhibitor. In certain embodiments, surgical resection of the tumor is performed after 11 days or within 84 days from the last administration of the PD-L1 inhibitor. In certain embodiments, surgical resection of the tumor is performed between 11 and 84 days after the last administration of the PD-L1 inhibitor. In certain embodiments, surgical resection of the tumor is performed between 11 and 42 days after the last administration of the PD-L1 inhibitor. In certain embodiments, surgical resection of the tumor is performed between 18 and 35 days, or between 21 and 35 days after the last administration of the PD-L1 inhibitor.

In certain embodiments, the anti-PD-L1 antibody is administered to a patient twice at a dose of 20 mg/kg at intervals of 10 to 18 days or every 2 weeks, prior to surgical resection of the tumor, and the surgical resection of the tumor is performed between 11 and 42 days after the last administration of the anti-PD-L1 antibody.

All patents and references cited herein are incorporated herein by reference in their entirety.

Hereinafter, the present disclosure will be described in more detail by way of the following examples. However, the following examples are intended solely to illustrate the present disclosure and the scope of the present disclosure is not limited thereto.

**Examples**

**Example 1. Clinical trial of IMC-001 as neoadjuvant therapy for treatment of resectable gastric cancer, esophageal cancer, and liver cancer**

This study is a phase 2 multi-cohort trial of the immune checkpoint inhibitor IMC-001 as a neoadjuvant therapy for the treatment of resectable localized gastric adenocarcinoma (GC), esophageal squamous cell carcinoma (ESCC), or hepatocellular carcinoma (HCC). IMC-001 is a fully human anti-PD-L1 recombinant monoclonal antibody of IgG1 type, comprising a heavy chain that comprises the amino acid sequence set forth in SEQ ID NO: 3, and a light chain that comprises the amino acid sequence set forth in SEQ ID NO: 4. For the amino acid sequence information of IMC-001, see Table 1 below.

[Table 1]

| Category | Sequence | SEQ ID NO: |
|---|---|---|
| Heavy chain variable domain | QMQLVQSGAEVKKPGSSVKVSCKASGGTFSSYAYS WVRQAPGQGLEWMGGIIPSFGTANYAQKFQGRVTI TADESTSTAYMELSSLRSEDTAVYYCARGPIVATITP LDYWGQGTLVTVSS | 1 |
| Light chain variable domain | SYVLTQPPSVSVAPGKTATIACGGENIGRKTVHWYQ QKPGQAPVLVIYYDSDRPSGIPERFSGSNSGNTATLT ISRVEAGDEADYYCLVWDSSSDHRIFGGGTKLTVL | 2 |
| Heavy chain | MEWSWVFLFFLSVTTGVHSQMQLVQSGAEVKKPG SSVKVSCKASGGTFSSYAYSWVRQAPGQGLEWMG GIIPSFGTANYAQKFQGRVTITADESTSTAYMELSSL RSEDTAVYYCARGPIVATITPLDYWGQGTLVTVSSA STKGPSVFPLAPSSKSTSGGTAALGCLVKDYFPEPV TVSWNSGALTSGVHTFPAVLQSSGLYSLSSVVTVPS SSLGTQTYICNVNHKPSNTKVDKRVEPKSCDKTHT CPPCPAPELLGGPSVFLFPPKPKDTLMISRTPEVTCV VVDVSHEDPEVKFNWYVDGVEVHNAKTKPREEQY NSTYRVVSVLTVLHQDWLNGKEYKCKVSNKALPA PIEKTISKAKGQPREPQVYTLPPSREEMTKNQVSLTC LVKGFYPSDIAVEWESNGQPENNYKTTPPVLDSDGS FFLYSKLTVDKSRWQQGNVFSCSVMHEALHNHYT QKSLSLSPGK | 3 |
| Light chain | MSVPTQVLGLLLLWLTDARCSYVLTQPPSVSVAPG KTATIACGGENIGRKTVHWYQQKPGQAPVLVIYYD SDRPSGIPERFSGSNSGNTATLTISRVEAGDEADYYC LVWDSSSDHRIFGGGTKLTVLGQPKAAPSVTLFPPS SEELQANKATLVCLISDFYPGAVTVAWKADSSPVKA GVETTTPSKQSNNKYAASSYLSLTPEQWKSHRSYSC QVTHEGSTVEKTVAPTECS | 4 |
| Heavy chain variable domain CDR1 | SYAYS | 5 |
| Heavy chain variable domain CDR2 | GIIPSFGTANYAQKFQG | 6 |

(continued)

| Category | Sequence | SEQ ID NO: |
|---|---|---|
| Heavy chain variable domain CDR3 | GPIVATITPLDY | 7 |
| Light chain variable domain CDR1 | GGENIGRKTVH | 8 |
| Light chain variable domain CDR2 | YDSDRPS | 9 |
| Light chain variable domain CDR3 | LVWDSSSDHRI | 10 |

IMC-001 is H6B1L-EM, which is a variant of the wild-type parent antibody H6B1L, and was produced in CHO cells. For more details, see International Publication No. WO 2017/132562 A1.

The study included the following cohorts: a cohort of patients with resectable gastric cancer; a cohort of patients with resectable esophageal cancer; and a cohort of patients with resectable hepatocellular carcinoma.

**Study Objectives**

An objective of this study is to evaluate the clinical activity of IMC-001 as neoadjuvant therapy in patients with lesions of resectable gastric cancer, esophageal cancer, and hepatocellular carcinoma.

**Rationale**

Neoadjuvant immunotherapy has potential biological benefits that include less immunosuppressed TME, lower disease burden, and antitumor immunity priming in the presence of primary tumor. Digestive cancers, including gastroesophageal cancer and hepatocellular carcinoma, are a type of cancer for which immune checkpoint inhibitors (ICIs) have shown survival benefit in palliative therapies. IMC-001, which is a fully human anti-PD-L1 recombinant monoclonal antibody that retains Fc effectors to stimulate ADCC, showed a favorable safety profile in the phase 1 study (n = 15) when administered intravenously at a dose of up to 20 mg/kg every 2 weeks while showing promising preliminary efficacy in patients with solid tumor who had been treated several times (1 case of partial response and 4 cases of stable disease) (Keam B, et al., Invest New Drugs 2021;39:1624-32).

**Study Endpoints**

The primary endpoint of this study is to evaluate a major pathological response rate in resected tissue following preoperative administration of the immune checkpoint inhibitor IMC-001. The secondary endpoint includes assessment of feasibility of delay in scheduled surgery and safety, assessment of R0 resection rate, assessment of the clinical tumor response rate according to RECIST v1.1, assessment of clinical disease control rate according to RECIST v1.1, assessment of progression-free survival, assessment of relapse-free survival, assessment of overall survival, and assessment of cancer progression/recurrence rate and cancer progression/recurrence pattern, with IMC-001 as preoperative therapy. Meanwhile, an exploratory objective of this study is to obtain specimens (tissue, blood, and feces) from subjects before and after administration of the immune checkpoint inhibitor IMC-001 in order to establish a biomarker cohort while performing immune profiling and genomic analysis in order to discover biomarkers predictive of therapeutic effects.

**Study Design**

The subjects who met the inclusion/exclusion criteria were assigned to the respective cohorts of gastric cancer, esophageal cancer, and hepatocellular carcinoma. In each cancer cohort group, the subjects received two cycles of IMC-001 (1 cycle = 2 weeks) as prior therapy, followed by curative surgery, and subsequent follow-up. After the curative surgery, adjuvant therapy was administered as appropriate according to standard practice guidelines based on the stage of the disease.

The study targeted patients who had not received treatment for resectable localized gastric cancer, esophageal cancer, or hepatocellular carcinoma, and a total of 48 patients (16 patients for each cancer cohort) were enrolled in the study.

**Neoadjuvant Therapy**

In the absence of unacceptable toxicity, clinical cancer progression, or withdrawal of consent, IMC-001 at 20 mg/kg was administered twice every 2 weeks as prior therapy before surgical resection.

**Biomarker Specimen Collection**

Tumor tissue, blood, and feces were collected before the administration of IMC-001. Blood and feces were collected after the first cycle and second cycle of administration. Resected tumor tissue, blood, and feces were collected following the surgery.

**Follow-up**

Starting from the date of surgery, blood, feces, and tumor tissue (only in the event of recurrence) were collected and observed every three months for the first two years, every six months for the subsequent three years, and at the time of cancer recurrence.

**Subjects**

This study enrolled a total of 48 patients (16 patients for each cancer cohort) with gastric adenocarcinoma, esophageal squamous cell carcinoma, or hepatocellular carcinoma, which is surgically resectable with curative intent.

**Inclusion Criteria**

Inclusion criteria for diseases

1) Histologically confirmed localized gastric adenocarcinoma, esophageal squamous cell carcinoma, hepatocellular carcinoma, or hepatocellular carcinoma clinically diagnosed according to the American Association for the Study of Liver Disease (AASLD) guidelines. However, for hepatocellular carcinoma that can be clinically diagnosed according to the AASLD guidelines, no biopsy is performed.

2) Gastric adenocarcinoma, esophageal squamous cell carcinoma, or hepatocellular carcinoma, which is expected to be amenable to curative resection

- Gastric adenocarcinoma: clinical stage $\geq$ T2 or lymph node metastasis-positive (AJCC 8th)
- Esophageal squamous cell carcinoma: clinical stage $\geq$ T1b or lymph node metastasis-positive (AJCC 8th)
- Hepatocellular carcinoma: Single hepatocellular carcinoma confined to the liver, or three or fewer hepatocellular carcinomas confined to the liver. Even if there is invasion of the portal vein, hepatic vein, or bile duct, invasion of the main portal trunk must not be present.

3) Hematology, blood chemistry, and major organ function requirements must meet the following criteria (to be confirmed within seven days prior to the first dose of the investigational drug).

- Absolute neutrophil count $\geq$ 1,000/$\mu$L
- Platelet count $\geq$ 75,000/$\mu$L
- Serum total bilirubin $\leq$ 1.5 x upper limit of normal (ULN) (for subjects with Gilbert syndrome, total bilirubin $\leq$ 3.0 x ULN)
- Aspartate aminotransferase (AST) or alanine aminotransferase (ALT) $\leq$ 2.5 x ULN; alkaline phosphatase $\leq$ 2.5 x ULN
- Serum creatinine $\leq$ 1.5 x upper limit of normal, or creatinine clearance $\geq$ 50 mL/minute (in which the creatinine clearance is first calculated using the Cockcroft-Gault formula below, based on gender. If the value is less than 50 mL/min, a 24-hour urine collection test is performed. Here, subjects whose creatinine clearance is 50 mL/min or greater are eligible for enrollment)
[Creatinine clearance for males]

$$\text{Clearance} = \frac{(140 - \text{Age [years]} \times \text{Body Weight}\,[kg])}{(72) \times (\text{Serum Creatinine}\,[mg/dL])}$$

[Creatinine clearance for females]

$$\text{Clearance} = \frac{(140 - \text{Age [years]} \times \text{Body Weight}\,[kg])}{(72) \times (\text{Serum Creatinine}\,[mg/dL])} \times 0.85$$

- Urine protein-creatinine ratio (UPC) ≤ 1 (however, subjects with UPC > 1 may be eligible for enrollment if a 24-hour urine protein test shows < 2 g)
- In addition, for hepatocellular carcinoma, subjects must have Child-Pugh class A (see Table 4) and grade 0 encephalopathy (see Table 3), with sufficient liver function confirmed (see Table 2).

[Table 2]

| Scoring | | | |
|---|---|---|---|
| Parameter | 1 Point | 2 Points | 3 Points |
| Total bilirubin (mg/dL) | <2.0 | 2.0-3.0 | >3.0 |
| Albumin (g/dL) | >3.5 | 2.8-3.5 | <2.8 |
| Prothrombin time (PT)[a] | | | |
| Prolonged time relative to control (sec) | <4.0 | 4.0-6.0 | >6.0 |
| International normalized ratio (INR) | <1.7 | 1.7-2.3 | >2.3 |
| Ascites | None | Mild | Moderate |
| Encephalopathy (grade) | None (Grade 0) | Mild to moderate (Grade 1/2) | Severe (Grade 3/4) |
| [a]Select whichever has a higher point: Prolonged time relative to control (sec) or INR outcome | | | |

[Table 3]

| Encephalopathy grading | |
|---|---|
| Grade | Clinical definition |
| 0 | Normal mental status, personality, and neurological examination |
| 1 | Restlessness, sleep disturbances, irritability/agitation, tremor, and handwriting impairment |
| 2 | Drowsiness, disorientation in time, inappropriate behavior, asterixis, and ataxia |
| 3 | Somnolence, stupor, impaired place recognition, hyperreflexia, rigidity |
| 4 | Coma without arousal, absence of personality/behavior, and loss of rationality |

[Table 4]

| Classification | |
|---|---|
| Points | Child-Pugh Class |
| 5-6 | A |
| 7-9 | B |
| 10-15 | C |

4) Measurable lesion or evaluable lesion disease according to the Response Evaluation Criteria in Solid Tumors (RECIST) version 1.1 (see Eisenhauer EA, Therasse P, Bogaerts J, Schwartz LH, Sargent D, Ford R, et al., New response evaluation criteria in solid tumours: revised RECIST guideline (Version 1.1). Eur J Cancer. 2009; 45:228 247).

5) Must be able to provide a tumor tissue specimen deemed suitable for biomarker analysis (for hepatocellular carcinoma, enrollment is possible even if no tumor tissue specimen is available prior to administration of the investigational drug).

General inclusion criteria

6) 19 years of age or older
7) Eastern Cooperative Oncology Group (ECOG) performance status of 0 to 1 (see Table 5)

[Table 5]

| | ECOG performance status |
|---|---|
| 0 | Fully active, able to carry on all pre-disease performance without restriction |
| 1 | Restricted in physically strenuous activity, but ambulatory and able to carry out work of a light and sedentary nature (for example, light house work, office work) |
| 2 | Ambulatory and capable of all self-care but unable to carry out any work activities. Up and about more than 50% of waking hours. |
| 3 | Capable of only limited self-care, confined to bed or chair more than 50% of waking hours |
| 4 | Completely disabled. Cannot carry on any self-care. Totally confined to bed or chair. |
| 5 | Dead |

8) Must sign the informed consent form
9) For both male and female subjects of reproductive potential, they must be able to comply with adequate contraceptive methods during the treatment period and for up to 3 months after the last dose of the investigational drug

**Exclusion Criteria**

Subjects who meet any of the following criteria were excluded from the clinical trial.

Tumor-related exclusion criteria

1) Unresectable or metastatic disease

2) Previous treatment for gastric adenocarcinoma, esophageal squamous cell carcinoma, or hepatocellular carcinoma. However, for hepatocellular carcinoma, even in a case where a subject has received prior treatment for a localized lesion, the subject may be eligible for clinical trial enrollment if more than 6 months have passed since the treatment, the treated area has progressed or a new lesion has developed outside the previously treated area, curative resection is feasible, and the subject meets the other inclusion/exclusion criteria.

3) Patients with a history of another cancer within 3 years prior to initiation of trial treatment. However, patients with cancer that is unlikely to affect prognosis, such as carcinoma in situ or papillary thyroid cancer, may be enrolled at the investigator's discretion.

4) History of hepatic encephalopathy

5) Clinically significant ascites, defined as follows:

- When screening, the physical examination reveals ascites or

- Previous ascites that required treatment and continuous prevention or current ascites that require treatment

Investigational drug-related exclusion criteria

6) History of active autoimmune disease, which requires systemic treatment (that is, disease-modifying agents, corticosteroids, or immunosuppressive agents), within the past two years. Replacement therapy (for example, physiological corticosteroid replacement therapy due to dysfunction of thyroxine or insulin, adrenal gland, or pituitary gland) is not considered as a form of systemic treatment and is permitted.

7) Subjects diagnosed with immunodeficiency or receiving chronic systemic steroid therapy (at a dose exceeding 10 mg/day of prednisone or equivalent) or any other form of immunosuppressive therapy within 7 days prior to the first dose of the investigational drug.

8) Subjects who have a history of (non-infectious) interstitial pneumonia that required steroid treatment or who currently have interstitial pneumonia.

9) Prior treatment with anti-PD-1, anti-PD-L1, anti-PD-L2, anti-CTLA-4 antibodies, or other antibodies or drugs that specifically target T cell co-stimulatory or checkpoint pathways.

10) Known severe hypersensitivity or anaphylaxis to recombinant proteins including monoclonal antibodies.

General exclusion criteria

11) Active infection that requires systemic treatment

12) Have a history of, or current evidence of any condition, therapy, or laboratory abnormality that might confound the results of the clinical trial, interfere with the participant's participation for the full duration of the clinical trial, or is not in the best interest of the participant to participate, in the opinion of the investigator

13) Female subjects of childbearing potential who have a positive urine or blood pregnancy test within 7 days prior to the first dose of the investigational drug

14) Female subjects who are pregnant or breastfeeding, or planning to become pregnant or have a child during the clinical trial period and up to 90 days after the last dose of the investigational drug

15) History or current evidence of symptomatic congestive heart failure (that is, New York Heart Association Classification > Class II) or clinically significant cardiac arrhythmia that requires treatment with antiarrhythmic drugs other than beta-blockers or digoxin, and/or onset of conduction abnormality (atrial fibrillation and paroxysmal ventricular tachycardia are exceptions), active coronary artery disease, unstable angina within 6 months prior to initiation of the clinical trial, new onset of angina within 3 months prior to enrollment of the clinical trial, or onset of myocardial infarction within 6 months prior to enrollment of the clinical trial

16) Known history of human immunodeficiency virus (HIV) infection (HIV 1/2 antibodies)

17) Subjects with known active hepatitis B (detectable hepatitis B surface antigen HbsAg or HBV DNA) or hepatitis C (detectable HCV RNA). However, subjects with hepatitis B may be eligible for enrollment if HBV DNA < 500 IU/mL (or 2500 copies/mL) at screening. Subjects with detectable HBsAg or detectable HBV DNA should be managed according to treatment guidelines. Subjects receiving antiviral drugs at screening must have been receiving treatment for at least 2 weeks prior to enrollment and must continue receiving treatment for 6 months after treatment with the investigational drug. Patients with positive HCV antibody may be eligible for enrollment only if they are HCV RNA negative in a PCR test (however, subjects with hepatocellular carcinoma are eligible for enrollment regardless of their HCV RNA positivity)

18) History of allogeneic tissue/solid organ transplantation

19) Received a live vaccine within 28 days prior to the first dose of the investigational drug

**Experimental Treatment (preoperative treatment with IMC-001)**

Method for preparing and administering investigational drug

IMC-001 was provided as an injectable, filled in 10 mL sterile glass vials at concentrations of 10 mg/mL and 60 mg/mL in a histidine-based solution. IMC-001 was diluted in 250 mL of 0.9% sodium chloride and administered as an intravenous infusion over 60 minutes (±10 minutes). Subjects were carefully monitored for any infusion-related reactions during the administration of IMC-001. Premedication was permitted, and premedication with antihistamines, antipyretics, and/or analgesics (for example, acetaminophen) could be carried out at the investigator's discretion. In the event of an infusion-related reaction, subjects were managed according to treatment guidelines. Vital signs (blood pressure, pulse rate, respiratory rate, and body temperature) were measured within 60 minutes prior to the infusion. If clinically indicated, vital signs were measured every 15 minutes (±10 minutes) during the infusion or at 30 minutes (±10 minutes) after the infusion. For subjects who experienced an infusion-related reaction during the first infusion, vital signs were measured during the infusion and at 30 minutes (±10 minutes) after the infusion.

Initial dose and dosing schedule

The dose of IMC-001 for this clinical trial is 20 mg/kg, which was determined in the ongoing phase 1 clinical trial, as a recommended dose (RP2D) for a phase 2 clinical trial. It was administered via an intravenous infusion on Day 1 (±4 days) of each 14-day cycle. Dose calculation was based on the weight assessed at baseline. If the subject's weight was within 10% of the weight used in the previous dose calculation, the dose was not recalculated. All doses are rounded to the nearest milligram (mg) according to the standards of the clinical trial site.

Total duration of administration

A total of two cycles of IMC-001 were administered prior to surgery unless subjects discontinued prematurely due to unacceptable toxicity or cancer progression, or a determination that continued treatment would be detrimental, or unless consent was withdrawn.

Surgery:

Surgery was performed within four weeks after completion of the second cycle of IMC-001 as preoperative therapy (at a time point between days 11 and 42 from the date of IMC-001 administration [Day 1] of the second cycle). However, the end of prior therapy visit preceded the date of the surgery. At this time, subjects had a white blood cell count greater than 3,000/μL, a platelet count greater than 75,000/μL, no Grade 2 or higher toxicity due to treatment with investigational drug and were in a clinical condition suitable for major surgery. Surgical procedures were performed according to institutional treatment guidelines for gastric cancer, esophageal cancer, and hepatocellular carcinoma. Open surgery was considered the standard approach. Laparoscopic and robot-assisted surgeries were permitted after consultation with the principal investigator only in a case of being performed by a surgical team with extensive professional experience, in which the laparoscopic and robot-assisted surgeries are considered equivalent to open surgery in terms of morbidity, mortality, and oncologic outcomes. Laparoscopic surgery for clinical trial could be considered by the surgical team during initial evaluation after reviewing the extent and resectability of the tumor and evidence of distant metastases.

Resection Surgery

The extent of surgical resection was determined based on the tumor's location, size, and progression. Resectability of the cancer was determined by the attending surgeon during the surgery. The following conditions could be considered reasons for unresectability:

- Distant metastasis

- Tumor invasion into major vascular structures and/or other organs

- Other cases in which curative resection is deemed unlikely

In general, if complete resection (R0) was not feasible, the decision to proceed with non-curative surgery was made by the surgical team.

Lymphadenectomy

The extent of lymphadenectomy was determined according to institutional treatment guidelines for gastric cancer, esophageal cancer, and hepatocellular carcinoma.

Postoperative treatment

Adjuvant chemotherapy following curative resection as well as treatment for subjects who underwent non-curative resection or were unable to undergo resection was administered according to institutional guidelines.

## Prohibited Concomitant Medications and Treatments

The following medications and treatments were prohibited during the clinical trial (except when used to treat adverse events related to the investigational drug).

- Any anticancer therapy, other than the investigational drug, for cancer treatment (including chemotherapy, hormone therapy, immunotherapy, radiotherapy, biological anticancer therapy, or traditional herbal medicine) during administration of the prior anticancer therapy in this clinical trial

- Any other investigational treatment within 28 days before the start of administration of the investigational drug used in this clinical trial and during administration of the investigational drug

- Live attenuated vaccines (for example, FluMist®) within 28 days before the start of administration of the investigational drug, during administration of the investigational drug, and for 5 months after the final dose of the investigational drug

- Systemic immunosuppressants (cyclophosphamide, azathioprine, methotrexate, thalidomide, and the like)

- Systemic corticosteroids: Use of corticosteroids at doses greater than or equivalent to 10 mg/day of prednisone was generally not permitted. Temporary use of corticosteroids exceeding 10 mg/day was permitted if medically necessary, such as for managing symptoms of infusion reactions or adverse events such as irAEs or when administered before computed tomography (CT) imaging in subjects with a history of allergies to intravenous contrast agents. If the dose of corticosteroids could not be reduced to a dose of 10 mg/day or less of prednisone within 12 weeks after the start of the steroids, the investigator promptly initiated a discussion regarding whether to continue treatment with the investigational drug for the subject.

## Permitted Concomitant Medications and Treatments

The following medications and treatments were permitted.

- Oral contraceptives

- Hormone-replacement therapy

- Inactivated influenza vaccination

- Megestrol acetate administered as an appetite stimulant

- Topical, ophthalmic, intra-articular, intranasal, or inhaled corticosteroids

- Adrenal replacement steroids, prednisone at < 10 mg/day

- Corticosteroids used temporarily for prevention (for example, of contrast agent allergy)

The use of other medications necessary for management of subjects was at the discretion of the investigator. In the event of nausea, vomiting, or diarrhea, effective symptomatic treatment was initiated. Antiemetics were provided to subjects according to local institutional guidelines or other guidelines (for example, ASCO guidelines). For treatment of febrile neutropenia, hematopoietic growth factors, that is, G-CSF or GM-CSF, could be used according to institutional or other guidelines (for example, ASCO guidelines); however, they were used for primary prophylaxis. For treatment of infusion reactions related to the investigational drug, paracetamol (acetaminophen), pethidine (meperidine), and chlorphenamine (chlorpheniramine) or other antihistamines were used according to institutional guidelines.

**Assessment of Adverse Event Severity**

The grading scale provided in the NCI Common Terminology Criteria for Adverse Events (NCI CTCAE) v4.03 (http://ctep.cancer.gov/protocolDevelopment/electronic_applications/ctc.htm#ctc_40) was used to assess severity of adverse events. For adverse events not listed in the NCI CTCAE, the criteria in Table 6 was used to assess severity. Heart failure was classified according to the NYHA classification criteria.

[Table 6]

| Severity grading scale for adverse events not specified in NCI CTCAE | | |
|---|---|---|
| Grade | Severity | Definition |
| 1 | Mild | Asymptomatic or mild symptoms; clinical or diagnostic observations only; intervention not indicated. |
| 2 | Moderate | Minimal, local or noninvasive intervention indicated; limiting age-appropriate instrumental ADL* |
| 3 | Severe or medically significant but not immediately life-threatening | Hospitalization or prolongation of hospitalization indicated; disabling; limiting self-care ADL† |
| 4 | Life-threatening consequences | Urgent intervention indicated |
| 5 | Death | Death related to AE |
| *Instrumental ADL refers to preparing meals, shopping for groceries or clothes, using the telephone, managing money, and the like.<br>†Self-care ADL refers to bathing, dressing and undressing, feeding self, using the toilet, taking medications, and not bedridden. | | |

**Example 2. Clinical trial results of IMC-001 as neoadjuvant therapy for treatment of resectable GC, ESCC, and HCC**

**Safety**

Except for G3 AST/ALT elevations (n = 3, 6%), all treatment-related adverse events (TRAEs) were G1 or G2. TRAEs that occurred in 5% or less of patients were hyperthyroidism (n = 9, 18%), hypothyroidism (n = 7, 14%), fatigue (n = 7, 14%), pruritus (n = 6, 12%), skin rash (n = 4, 8%), infusion-related reactions (n = 3, 6%), and diarrhea (n = 3, 6%).

**Clinical Tumor Response**

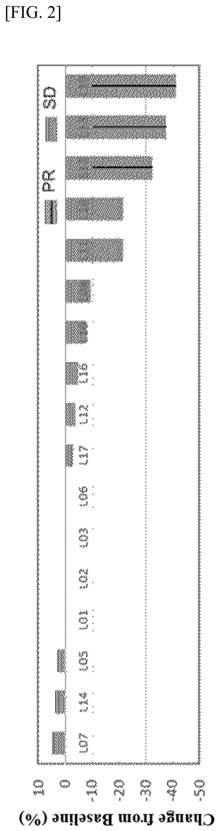

No patients experienced disease progression during the neoadjuvant therapy. Among the 17 patients with measurable lesions according to RECIST 1.1, 3 (17.6%) had partial response (PR), and 14 (82.4%) had stable disease (SD) (FIG. 2).

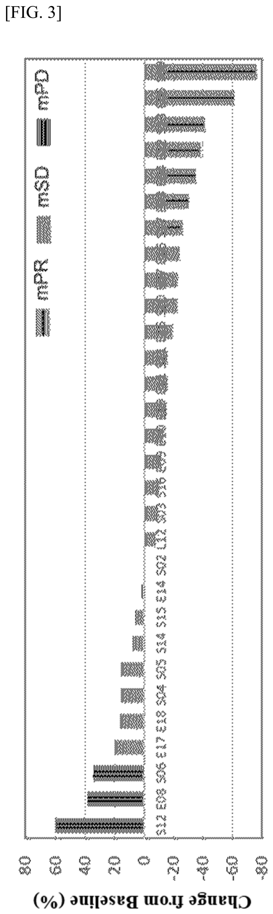

Among the 30 patients evaluable for metabolic response based on EORTC criteria, 7 (23.3%) had metabolic partial response (mPR), 20 (66.7%) had metabolic stable disease (mSD), and 3 (10.0%) had metabolic progressive disease (mPD) (FIG. 3). Two of the three patients with metabolic progression disease had increased tumor-infiltrating lymphocytes and tumor regression in surgical pathology.

**Surgical Results**

All patients underwent curative R0 resection with pathological stage I (n = 36, 75.0%)/II (n = 6, 12.5%)/III (n = 6, 12.5%). There were no surgical mortalities or treatment-related surgical morbidities.

**Pathologic Response**

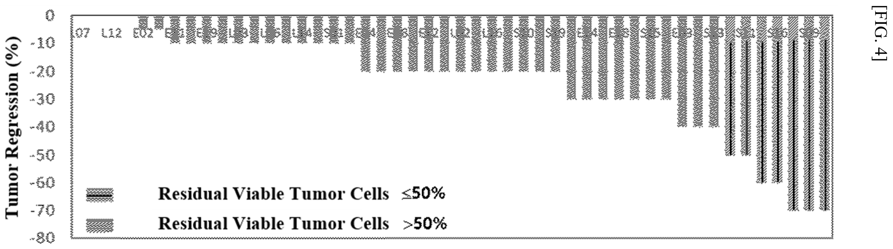

Although there were no major pathological responses, 51% of the patients showed varying degrees of tumor necrosis or fibrosis, and 7 patients (15%) had less than 50% of residual viable tumor cells (FIG. 4).

**Recurrence**

With a median follow-up duration of 31.4 months (range: 1.7 to 41.2 months), recurrence occurred in only 4 patients (8.3%). The median progression-free survival (PFS) or recurrence-free survival (RFS) was not reached.

**Conclusion**

Neoadjuvant therapy with IMC-001 exhibited good tolerability and promising antitumor activity in patients with resectable, microsatellite-stable gastric cancer (GC), esophageal cancer (EC), and hepatocellular carcinoma (HCC).

**Claims**

1. A pharmaceutical composition for treating a tumor or inhibiting tumor proliferation in a patient with upper gastrointestinal cancer, comprising:

   a therapeutically effective amount of an anti-PD-L1 antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2,
   wherein the antibody is administered to the patient as neoadjuvant therapy prior to the surgical resection of the tumor in the patient.

2. The pharmaceutical composition of claim 1, wherein the upper gastrointestinal cancer is gastric cancer, esophageal cancer, or liver cancer.

3. The pharmaceutical composition of claim 1, wherein the anti-PD-L1 antibody comprises a heavy chain variable region comprising HCDR1 of SEQ ID NO: 5, HCDR2 of SEQ ID NO: 6, and HCDR3 of SEQ ID NO: 7, and a light chain variable region comprising LCDR1 of SEQ ID NO: 8, LCDR2 of SEQ ID NO: 9, and LCDR3 of SEQ ID NO: 10.

4. The pharmaceutical composition of claim 3, wherein the light chain variable region comprises the amino acid sequence of SEQ ID NO: 2, or comprises an amino acid sequence that is at least 95% identical to SEQ ID NO: 2.

5. The pharmaceutical composition of claim 4, wherein the amino acids at positions 3 and 5 of the light chain variable region are identical to the amino acids at positions 3 and 5 of SEQ ID NO: 2.

6. The pharmaceutical composition of claim 4, wherein the heavy chain variable region comprises an amino acid sequence that is at least 95% identical to SEQ ID NO: 1.

7. The pharmaceutical composition of claim 1, wherein the anti-PD-L1 antibody comprises a heavy chain having the amino acid sequence of SEQ ID NO: 3 and a light chain having the amino acid sequence of SEQ ID NO: 4.

8. The pharmaceutical composition of claim 1, wherein the upper gastrointestinal cancer is resectable.

9. The pharmaceutical composition of claim 1, wherein the upper gastrointestinal cancer is recurrent.

10. The pharmaceutical composition of claim 1, wherein the upper gastrointestinal cancer is metastatic.

11. The pharmaceutical composition of claim 1, wherein the upper gastrointestinal cancer is surgically treated with curative intent.

12. The pharmaceutical composition of claim 2, wherein the gastric cancer is gastric submucosal tumor or gastric adenocarcinoma (GC).

13. The pharmaceutical composition of claim 2, wherein the esophageal cancer is esophageal squamous cell carcinoma (ESCC).

14. The pharmaceutical composition of claim 2, wherein the liver cancer is hepatocellular carcinoma (HCC).

15. The pharmaceutical composition of claim 1, wherein the neoadjuvant therapy is such that the anti-PD-L1 antibody is administered in one or more doses prior to the surgical resection of the tumor.

16. The pharmaceutical composition of claim 15, wherein each of the doses is administered at intervals of 10 to 18 days.

17. The pharmaceutical composition of claim 16, wherein the neoadjuvant therapy is such that the anti-PD-L1 antibody is administered 2 to 6 times at intervals of 10 to 18 days prior to the surgical resection of the tumor.

18. The pharmaceutical composition of claim 1, wherein the neoadjuvant therapy is such that the anti-PD-L1 antibody is administered at a dose of 10 mg/kg to 30 mg/kg.

19. The pharmaceutical composition of claim 18, wherein the neoadjuvant therapy is such that the anti-PD-L1 antibody is administered at a dose of 20 mg/kg.

20. The pharmaceutical composition of claim 1, wherein the surgical resection of the tumor is performed between 11 and 84 days after the last administration of the anti-PD-L1 antibody.

21. A method for treating a tumor or inhibiting tumor growth, comprising:

    (a) selecting a patient with upper gastrointestinal cancer;
    (b) administering a therapeutically effective amount of a PD-L1 inhibitor to the patient; and
    (c) surgically resecting a tumor of the upper gastrointestinal cancer after step (b),

    wherein the PD-L1 inhibitor is an antibody that specifically binds to PD-L1 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2.

22. A kit for treating a tumor or inhibiting tumor proliferation in a patient with upper gastrointestinal cancer, comprising a PD-L1 inhibitor together with a manual for using the PD-L1 inhibitor as neoadjuvant therapy,
    wherein the manual comprises instructions for intravenously administering the PD-L1 inhibitor 2 to 6 times at a dose of 20 mg/kg at intervals of 10 to 18 days prior to surgical resection of the tumor.

[FIG. 1]

[FIG. 2]

[FIG. 3]

[FIG. 4]

## INTERNATIONAL SEARCH REPORT

| International application No. |
|---|
| **PCT/KR2024/004990** |

| A. | CLASSIFICATION OF SUBJECT MATTER |
|---|---|

**C07K 16/28**(2006.01)i; **A61P 35/00**(2006.01)i; **A61K 39/00**(2006.01)i

According to International Patent Classification (IPC) or to both national classification and IPC

| B. | FIELDS SEARCHED |
|---|---|

Minimum documentation searched (classification system followed by classification symbols)

C07K 16/28(2006.01); A61K 39/00(2006.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 면역항암제 (cancer immunotherapy), 신보조요법 (neoadjuvant therapy), 면역관문 억제제 (immune checkpoint inhibitor), PD-L1 (programmed death-ligand 1), 항 PD-L1 항체 (anti-PD-L1 antibody), 상부 소화기암 (upper gastrointestinal cancer), 위암 (gastric cancer), 간암 (liver cancer), 외과적 절제 (surgical resection)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2017-0135860 A (CYTOMX THERAPEUTICS, INC.) 08 December 2017 (2017-12-08)<br>See claim 81; paragraphs [0004] and [0261]; and SEQ ID NOs: 1122 and 1138. | 1-6,8-20,22 |
| A | | 7 |
| Y | ZHANG, X. et al. Neoadjuvant PD-1 blockade plus chemotherapy versus chemotherapy alone in locally advanced stage II-III gastric cancer: A single-centre retrospective study. Translational Oncology. 17 March 2023, vol. 31, article no. 101657, inner pp. 1-9.<br>See abstract; and inner page 2. | 1-6,8-20,22 |
| A | PETRICEVIC, B. et al. Neoadjuvant immunotherapy in gastrointestinal cancers - The new standard of care?. Seminars in Cancer Biology. 2022, vol. 86, pp. 834-850.<br>See abstract; pages 834-844; and figures 1 and 2. | 1-20,22 |
| A | KR 10-2019-0026642 A (SORRENTO THERAPEUTICS, INC.) 13 March 2019 (2019-03-13)<br>See abstract; and claims 1-16. | 1-20,22 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 January 2025** | **09 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/004990** |

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2019-0088986 A (SHANGHAI HENLIUS BIOTECH, INC.) 29 July 2019 (2019-07-29)<br>See abstract; and claims 1-34. | 1-20,22 |
| X | PARK, S. R. et al. A phase II study of neoadjuvant immune checkpoint inhibitor IMC-001 (anti-PD-L1 antibody) in patients with resectable upper gastrointestinal cancers (Neo-Chance Study). In: AACR Annual Meeting. Cancer Research. 14 April 2023, vol. 83, 8_Supplement, abstract no. CT147.<br>See abstract.<br>(This document is a document declaring 'exceptions to lack of novelty' by the applicant.) | 1-20,22 |
| X | Medical Times. 이문온시아, AACR에서 수술전 요법 가능성 제시. 18 April 2023, non-official translation (ImmuneOncia Presents Potential Preoperative Therapy at AACR). Retrieved from <https://www.medicaltimes.com/Users/News/NewsView.html/%27http:/www.medigatenews.com/Users2/News/NewsView.html?ID=1153140>.<br>See entire document.<br>(This document is a document declaring 'exceptions to lack of novelty' by the applicant.) | 1-20,22 |

Form PCT/ISA/210 (second sheet) (July 2022)

## INTERNATIONAL SEARCH REPORT

| International application No. |
| --- |
| **PCT/KR2024/004990** |

**Box No. I      Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/004990** |

---

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **21**
   because they relate to subject matter not required to be searched by this Authority, namely:

   Claim 21 pertains to a method for treatment of the human body by surgery or therapy, as well as a diagnostic method (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

Form PCT/ISA/210 (continuation of first sheet) (July 2022)

| INTERNATIONAL SEARCH REPORT | | International application No. |
|---|---|---|
| Information on patent family members | | PCT/KR2024/004990 |

| Patent document cited in search report | | | Publication date (day/month/year) | Patent family member(s) | | | Publication date (day/month/year) |
|---|---|---|---|---|---|---|---|
| KR | 10-2017-0135860 | A | 08 December 2017 | AU | 2016-233495 | A1 | 26 October 2017 |
| | | | | BR | 11-2017-019559 | A2 | 15 May 2018 |
| | | | | CA | 2978942 | A1 | 22 September 2016 |
| | | | | CN | 108112254 | B | 28 January 2022 |
| | | | | CN | 114702586 | A | 05 July 2022 |
| | | | | EA | 2017-92032 | A1 | 30 April 2018 |
| | | | | EP | 3268392 | A2 | 17 January 2018 |
| | | | | IL | 292449 | B2 | 01 February 2024 |
| | | | | JP | 2023-002706 | A | 10 January 2023 |
| | | | | MY | 194225 | A | 22 November 2022 |
| | | | | NZ | 736142 | A | 26 November 2021 |
| | | | | NZ | 773949 | A | 22 March 2024 |
| | | | | SG | 10-2019-13297 | A | 27 February 2020 |
| | | | | SG | 11-2017-07383 | A | 30 October 2017 |
| | | | | US | 10669339 | B2 | 02 June 2020 |
| | | | | US | 2020-231677 | A1 | 23 July 2020 |
| | | | | US | 2022-298245 | A1 | 22 September 2022 |
| | | | | WO | 2016-149201 | A2 | 22 September 2016 |
| KR | 10-2019-0026642 | A | 13 March 2019 | AU | 2017-212717 | A1 | 03 August 2017 |
| | | | | BR | 11-2018-015480 | A2 | 21 May 2019 |
| | | | | CA | 3013051 | A1 | 03 August 2017 |
| | | | | CL | 2018-002034 | A1 | 15 March 2019 |
| | | | | CN | 109715821 | B | 06 September 2022 |
| | | | | EA | 2018-91709 | A1 | 31 January 2019 |
| | | | | EP | 3408400 | C0 | 26 June 2024 |
| | | | | IL | 260846 | A | 20 September 2018 |
| | | | | JP | 2022-095910 | A | 28 June 2022 |
| | | | | TW | 2017-36400 | A | 16 October 2017 |
| | | | | TW | I760323 | B | 11 April 2022 |
| | | | | US | 11919952 | B2 | 05 March 2024 |
| | | | | US | 2021-147539 | A1 | 20 May 2021 |
| | | | | WO | 2017-132562 | A1 | 03 August 2017 |
| KR | 10-2019-0088986 | A | 29 July 2019 | AU | 2017-348475 | B2 | 08 February 2024 |
| | | | | BR | 11-2019-008634 | A2 | 09 July 2019 |
| | | | | CA | 3041078 | A1 | 03 May 2018 |
| | | | | CN | 109963589 | A | 02 July 2019 |
| | | | | CN | 109963589 | B | 05 May 2023 |
| | | | | CN | 117088979 | A | 21 November 2023 |
| | | | | EP | 3532100 | A1 | 04 September 2019 |
| | | | | JP | 2019-535254 | A | 12 December 2019 |
| | | | | JP | 7011657 | B2 | 10 February 2022 |
| | | | | RU | 2019-116661 | A | 30 November 2020 |
| | | | | TW | 2018-23273 | A | 01 July 2018 |
| | | | | US | 11618786 | B2 | 04 April 2023 |
| | | | | US | 2019-225691 | A1 | 25 July 2019 |
| | | | | US | 2022-144955 | A1 | 12 May 2022 |
| | | | | WO | 2018-080812 | A1 | 03 May 2018 |
| | | | | ZA | 2019-02533 | B | 29 July 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7087411 B
- US 6054297 A
- US 5886152 A
- US 5877293 A
- WO 2017132562 A1
- US 5648237 A
- US 5789199 A
- US 5840523 A
- US 4816567 A
- US 4946778 A
- US 8257740 B
- US 8246995 B
- US 20190040137 A

### Non-patent literature cited in the description

- **PARDOLL DM**. The blockade of immune checkpoints in cancer immunotherapy. *Nat Rev Cancer*, 2012, vol. 12, 252-64
- **MCGRANAHAN N** ; **FURNESS AJ** ; **ROSENTHAL R et al.** Clonal neoantigens elicit T cell immunoreactivity and sensitivity to immune checkpoint blockade. *Science*, 2016, vol. 351, 1463-9
- **JAMAL-HANJANI M** ; **QUEZADA SA** ; **LARKIN J et al.** Translational implications of tumor heterogeneity. *Clin Cancer Res*, 2015, vol. 21, 1258-66
- **GIL DEL ALCAZAR CR** ; **HUH SJ** ; **EKRAM MB et al.** Immune Escape in Breast Cancer During In Situ to Invasive Carcinoma Transition. *Cancer Discov*, 2017, vol. 7, 1098-1115
- **TAYLOR et al.** *Nucl. Acids Res.*, 1992, vol. 20, 6287-6295
- Monoclonal Antibodies, Hybridomas: A New Dimension in Biological Analyses. Plenum Press, 1980
- Antibodies: A Laboratory Manual. Cold Spring Harbor Laboratory Press, 1988
- **MAYFIELD et al.** *Proc. Natl. Acad. Sci. USA.*, 2003, vol. 100 (2), 438-42
- **SINCLAIR et al.** *Protein Expr. Purif.*, 2002 (1), 96-105
- **CONNELL N D**. *Curr. Opin. Biotechnol.*, 2001, vol. 12 (5), 446-9
- **MAKRIDES et al.** *Microbiol. Rev.*, 1996, vol. 60 (3), 512-38
- **SHARP et al.** *Yeast.*, 1991, vol. 7 (7), 657-78
- **SAMBROOK et al.** Molecular Cloning: A Laboratory Manual. Cold Spring Harbor Laboratory Press, 1989, vol. 1-3
- **F. AUSUBEL et al.** Current Protocols in Molecular Biology. Green Publishing and Wiley-Interscience, 1987
- Cloning Vectors: A Laboratory Manual. Elsevier, 1985
- **CHARLTON**. Methods in Molecular Biology. Humana Press, 2003, vol. 248, 245-254
- **GLUZMAN et al.** *Cell*, 1981, vol. 23, 175
- **MCMAHAN et al.** *EMBO J.*, 1991, vol. 10, 2821
- **POUWELS et al.** Cloning Vectors: A Laboratory Manual. Elsevier, 1985
- **GRAHAM et al.** *J. Gen Virol.*, 1977, vol. 36, 59
- **MATHER**. *Biol. Reprod.*, 1980, vol. 23, 243-251
- **MATHER et al.** *Annals N.Y. Acad. Sci.*, 1982, vol. 383, 44-68
- **URLAUB et al.** *Proc. Natl. Acad. Sci. USA*, 1980, vol. 77, 4216
- **YAZAKI** ; **WU**. Methods in Molecular Biology. Humana Press, 2003, vol. 248, 255-268
- **GERNGROSS**. *Nat. Biotech.*, 2004, vol. 22, 1409-1414
- Solid Phase Peptide Synthesis. The Pierce Chemical Co, 1984
- **KORTT et al.** *Prot. Eng.*, 1997, vol. 10, 423
- **KORTT et al.** *Biomol. Eng.*, 2001, vol. 18, 95-108
- **KRIANGKUM et al.** *Biomol. Eng.*, 2001, vol. 18, 31-40
- **BIRD**. *Science*, 1988, vol. 242, 423
- **HUSTON et al.** *Proc. Natl. Acad. Sci. USA*, 1988, vol. 85, 5879
- **WARD et al.** *Nature*, 1989, vol. 334, 544
- **GRAAF et al.** *Methods Mol. Biol.*, 2002, vol. 178, 379-87
- **LANTTO et al.** *Methods Mol. Biol.*, 2002, vol. 178, 303-16
- **BLOOM et al.** *Protein Science*, 1997, vol. 6, 407
- Remington's Pharmaceutical Sciences. Mack Publishing Company

- **POWELL et al.** Compendium of excipients for parenteral formulations. *J Pharm Sci Technol*, 1998, vol. 52, 238-311
- **WU et al.** *J. Biol. Chem.*, 1987, vol. 262, 4429-4432
- **LANGER**. *Science*, 1990, vol. 249, 1527-1533
- **ARRUEBO, M. et al.** Antibody-conjugated nanoparticles for biomedical applications. *J. Nanomat.*, 2009, vol. 2009, 24
- **KEAM B et al.** *Invest New Drugs*, 2021, vol. 39, 1624-32
- **EISENHAUER EA** ; **THERASSE P** ; **BOGAERTS J** ; **SCHWARTZ LH** ; **SARGENT D** ; **FORD R et al.** New response evaluation criteria in solid tumours: revised RECIST guideline (Version 1.1). *Eur J Cancer.*, 2009, vol. 45, 228-247